# EUROPEAN PATENT SPECIFICATION

(11) **EP 1 916 010 B1**
(45) Date of publication and mention of the grant of the patent: **08.07.2009**
(21) Application number: 08150787.3
(22) Date of filing: 15.01.2002
(51) Int. Cl.: A61M 39/28

(54) **Flow rate controller**
Flussratenregler
Contrôleur de débit

(30) Priority: 15.01.2001 JP 2001007033; 30.03.2001 JP 2001101943; 03.04.2001 JP 2001105205; 28.06.2001 JP 2001197409
(43) Date of publication of application: 30.04.2008
(62) Divisional of application: 06118610.2
(73) Proprietor: TERUMO KABUSHIKI KAISHA, Tokyo 151-0072 (JP)
(72) Inventor: Yokota, Takayuki, Nakakoma-gun Yamanashi 409-3853 (JP); Matsumoto, Atsushi, Ashigarakami-gun Kanagawa 259-0151 (JP)
(74) Representative: TBK-Patent

(56) References cited:
- DE-A1- 19 712 908

## Description

### TECHNICAL FIELD

The present invention relates to a flow rate switcher for adjusting the flow rate of a liquid (fluid) such as liquid medicine, blood or liquid food, and to a medical instrument for liquid medication devices such as a fluid infusion set, blood transfusion set or nutritional transfusion set, using such flow rate devices.

### BACKGROUND ART

Flow rate adjustment of the liquid (fluid) within the tube, such as liquid medicine, blood and liquid food, is needed when using liquid medication devices, such as a fluid infusion set, blood transfusion set, and nutritional transfusion set. A flow rate controller, a flow rate regulator or a flow rate switcher is mounted at some midpoint of the tube for this purpose, as is well known in the art.

A typical example of such a flow rate controller, a flow rate regulator or a flow rate switcher is a roller type klemme (roller klemme) as disclosed, for example, in JP 58-22224 B. This roller klemme is composed of a klemme body and a movable roller attached to the klemme body. A tube is held between outer peripheral face of the roller and moderately inclined klemme body base. The roller is moved to change the inner diameter of the tube to regulate flow rate within the tube.

By using this roller klemme, in the case of a fluid infusion set for example, a medical operator visually checks the intravenous dripping speed in the tube and adjusts it to the required flow rate. However, it needed much caution because visual checking of the dripping speed was the only guideline to adjust flow rate.

Furthermore, as this roller klemme method utilizes entire inner peripheral of the tube to create a very small sectional area, this area tends to change with a passage of time due to deformation of squeezed soft tube, and so the properly adjusted flow rate of a liquid may change gradually. Flow rate adjusted around 100 ml/hr or less, in particular, needed burden of frequent rechecking of the flow rate.

Difference between targeted and actual flow rate is undesirable when treating a patient, and maintaining adjusted flow rate is especially required if the inflow of medicine per unit of time is restricted, such as anesthetic, antibiotic, cardiotonic, vasopressor and insulin. Moreover, as roller position is difficult to check with roller klemme, sometimes it is hard to determine whether change in flow rate is due to initial miss setting or patients' touching the devices.

Sometimes, a large quantity of infusion liquid is flown for a short time (flushing) after flow rate is adjusted. With roller klemme, roller must be shifted to end corner of klemme to give a big flow and re-shifted to its original position for an adjusted flow rate. Even in this case, burden of visual measurement and slight adjustment of roller position is needed as 1mm roller movement changes flow rate greatly. This arises a problem that re-adjusting after flashing is complicated.

The object of the present invention is to provide a flow rate switcher, which can easily adjust the flow rate to a target level, restrains the flow rate to the adjusted level with advance of time, and can easily be re-adjusted after temporary flushing, thus giving a sense of ease to medical operators.

The object of present invention is also to provide liquid medication units using this flow rate switcher.

In the present disclosure there are described the following items.
(1) :A flow rate controller comprising:
   a support body; an operating member slidably engaged with the support body; a cylindrical body situated between the support body and the operating member with a part of its outer periphery supported by the support body, having an inlet and an outlet for a fluid; and a flow passage control member formed of a hard material and in close contact with and in part closely fixed to the support body side of the interior of the cylindrical body forming a flow passage for the fluid; wherein: the operating member has a pressurizing portion to pressurize the flow passage side of outer peripheral portion of the cylindrical body; the cylindrical body or the flow passage control member has a control groove extending in the axial direction of the cylindrical body; and the sectional configuration of both side end portions of the flow passage control member in the vicinity of the boundary between the cylindrical body and the flow passage control member exhibits a continuous, gentle roundness.
(2) A flow rate controller comprising: a cylindrical operating member having a pressurizing portion and a supporting portion; a cylindrical body which is held between the pressurizing portion and the supporting portion and which has an inlet and an outlet for a fluid; and a flow passage control member formed of a hard material and in close contact with, and in part closely fixed to, the support body side of the interior of the cylindrical body forming a flow passage for the fluid; wherein:
   the operating member is engaged so as to be axially movable on the outer periphery of the cylindrical body; the pressurizing portion is provided on the operating member so as to be capable of pressurizing the flow passage side of outer peripheral portion of the cylindrical body; the cylindrical body or the flow passage control member has a control groove extending in the axial direction of the cylindrical body; and the section configuration of the side end portions of the flow passage control member in the vicinity of the boundary, between the cylindrical body and the flow passage control member is a continuous, gently rounded configuration.
(3) A flow rate controller as described in the item (1) or (2) wherein the control groove is provided in a flow passage surface of the flow passage control member.
(4) A flow rate controller as described in any one of the items (1) to (3), wherein the sectional area of the control groove increases or decreases continuously.
(5) A flow rate controller as described in any one of the items (1) to (4), wherein the surface configuration of the support body at the fixing position of the support body and the cylindrical body, and the surface configuration of the flow passage control member at the close fixing position of the cylindrical body and the flow passage control member are analogous.
(6) A flow rate controller as described in any one of the items (1) to (5), wherein: the flow passage control member has a concave section configuration to the axial direction of the cylindrical body on the flow passage side: and the pressurizing portion has a convex section configuration of a size which allows insertion into the concave configuration.
(7) A flow rate controller as described in any one of the items (1) to (6), wherein the radius R of the continuous gently rounded section configuration of the side end portions of the flow passage control member, and the thickness D of the cylindrical body are in the relationship: R ≥ D/10.
(8) A flow rate controller as described in any one of the items (1) to (7), wherein the cylindrical body has a deform portion adapted to be deformed where pressurized at the time of pressurization; and the deformed deform portion and the flow passage control member are in close contact with each other excluding the control groove.
(9) A flow rate controller as described in any one of the items (1) to (8), wherein the deform portion is constantly under a tensile strength applied in the inner section of the deformed portion to the axial direction of the cylindrical body.
(10) A flow rate controller as described in any one of the items (1) to (9), wherein in a non-pressurized state, the section configuration of the deform portion to the axial direction of the cylindrical body is linear.
(11) A flow rate controller as described in any one of the items (1) to (10), wherein the portion in close contact with the close contacted fixed portion of the interior of the cylindrical body and the flow passage control member is held in close contact without being fixed.
(12) A flow rate controller as described in any one of the items (1) to (11), wherein a friction reduction treatment is performed on the surface of the cylindrical body pressurized by the pressurizing portion.
(13) A flow rate controller as described in any one of the items (1) to (12), wherein the cylindrical body is composed of either one of, a fluid infusion tube, blood transfusion tube or nutritional transfusion tube.
(14) A flow rate controller as described in any one of the items (1) to (13), wherein inner peripheral length of the cylindrical body is smaller than cross sectional peripheral length of the flow passage control member excluding the control groove.
(15) A flow rate controller as described in the item (14), wherein the inner peripheral length L of the cylindrical body, and the cross sectional peripheral length M of the flow passage control member have following relationship: 0.6 < L/M < 1.
(16) A flow rate controller comprising:
   a support body; an operating member slidably engaged with the support body; a cylindrical member situated between the support body and the operating member having an inlet and an outlet for a fluid; wherein:
      the cylindrical member is composed of a flow passage formation control portion made of hard material fixed to the support member on the support member side, and a membrane-like portion made of a soft material on the operating member side; the operating member has a pressurizing portion to pressurize the membrane-like portion; the membrane-like portion or the flow passage formation control portion has a control groove extending in the axial direction; and the sectional configuration of both side end portions of the flow passage formation control portion in the vicinity of the boundary between the membrane-like portion and the flow passage formation control portion is a continuous, gently rounded configuration.
(17) A flow rate controller as described in the item (16), wherein the control groove is provided in a flow passage surface of the flow passage formation control portion.
(18) A flow rate controller as described in the item (16) or (17), wherein the sectional area of the control groove increases or decreases continuously.
(19) A flow rate controller as described in any one of items (16) to (18), wherein the flow passage formation control portion has a concave section configuration to the axial direction of the cylindrical body on the flow passage side; and the pressurizing portion has a convex configuration of a size that allows insertion into the concave configuration.
(20) A flow rate controller as described in any one of items (16) to (19), wherein the radius R of the continuous, gently rounded section configuration of the side end portions of the flow passage formation control portion, and the thickness D of the cylindrical body have following relationship: R ≥ D/10.
(21) A flow rate controller as described in any one of items (16) to (20), wherein the membrane-like portion has a deform portion adapted to be deformed where pressurized at the time of pressurization; and the deformed deform portion and the flow passage formation control portion are in close contact with each other excluding the control groove.
(22) A flow rate controller as described in any one of items (16) to (21), wherein the deform portion is constantly under tensile strength in the inner section to the axial direction of the cylindrical body.
(23) A flow rate controller as described in any one of items (16) to (22), wherein in a non-pressurized state, the section configuration of the deform portion to the axial direction of the membrane-like portion is linear.
(24) A flow rate controller as described in any one of items (16) to (23), wherein the supporting body and the flow passage formation control portion are integrally molded.
(25) A flow rate controller as described in any one of items (16) to (24), wherein friction reduction treatment is performed on the surface of the membrane-like portion pressurized by the pressurizing portion.
(26) A flow rate controller as described in any one of items (1) to (25), wherein:
   the operating member has an operating portion to make it move; and the operating portion is located in opposite side to the direction of pressurizing force made by the pressurizing portion.
(27) A flow rate controller as described in any one of items (1) to (26), wherein it has a scale for indicating the flow rate according to a displacement amount of the pressurizing portion.
(28) A flow rate regulator comprising:
   a pressurizing portion; a support portion; a cylindrical operating member having a pair of wall portions that are opposed to each other and elastically deformable; a cylindrical body which is sandwiched between the pressurizing portion and the support portion and has an inlet and an outlet for a fluid; and a flow passage bottom member which is formed of a hard material and in close contact with the support portion side of an interior of the cylindrical body forming a flow passage for the fluid and a part of which is closely fixed thereto; the flow rate regulator being wherein:
      the cylindrical body or the flow passage bottom member has a control groove extending in an axial direction of the cylindrical body; the operating member is engaged so as to be axially movable on an outer periphery of the cylindrical body; and the pressurizing portion is disposed so as to be capable of pressurizing the flow passage side outer peripheral portion of the cylindrical body and capable of canceling a pressurized state by pressurizing the pair of wall portions.
(29) A flow rate regulator comprising:
   a support body; a support member having a pair of wall portions that are opposed to each other and elastically deformable; an operating member slidably engaged with the pair of wall portions; a cylindrical body situated between the support member and the operating member, whose outer periphery is partially supported by the support member, and has an inlet and an outlet for a fluid; and a flow passage bottom member which is formed of a hard material and in close contact with the support body side of an interior of the cylindrical body forming a flow passage for the fluid and a part of which is closely fixed thereto; wherein:
      the cylindrical body or the flow passage bottom member has a control groove extending in an axial direction of the cylindrical body; the operating member has a pressurizing portion for pressurizing the flow passage side outer peripheral portion of the cylindrical body; and the pressurizing portion being capable of canceling a pressurized state of the cylindrical body by pressurizing the pair of wall portions.
(30) A flow rate regulator comprising:
   a pressurizing portion; a support portion; a cylindrical operating member having a pair of wall portions that are opposed to each other and elastically deformable; and a cylindrical member which is sandwiched between the pressurizing portion and the support body and has an inlet and an outlet for a fluid; wherein:
      the cylindrical member comprising of a flow passage bottom member formed of a hard material and a membrane-like portion which is on the operating member side and is formed of a soft material; the membrane-like portion or the flow passage bottom portion has a control groove extending in an axial direction of the cylindrical member; the operating member is engaged so as to be axially movable on an outer periphery of the cylindrical member; and the pressurizing portion is disposed so as to be capable of pressurizing the membrane-like portion and is capable of canceling a pressurized state by pressurizing the pair of wall portions.
(31) A flow rate regulator comprising:
   a support body; a support member having a pair of wall portions that are opposed to each other and elastically deformable; an operating member slidably engaged with the support body; and a cylindrical member situated between the support body and the operating member and has an inlet and an outlet for a fluid; wherein:
      the cylindrical member is composed of a flow passage bottom portion which is formed of a hard material fixed to the support portion and a membrane-like portion which is on the operating member side and is formed of a soft material; the membrane-like portion or the flow passage bottom portion has a control groove extending in an axial direction of the cylindrical member; and the operating member has a pressurizing portion for pressurizing the membrane-like portion; and the pressurizing portion being capable of canceling a pressurized state of the membrane-like portion by pressurizing the pair of wall portions.
(32) A flow rate regulator as described in any one of items (28) to (31), wherein: the pair of wall portions are each formed into a convex arch projecting outward; a sectional area of the control groove either gradually increases or decreases continuously; the flow passage bottom member has a concave section configuration as taken perpendicular to the axial direction of the cylindrical body or the cylindrical member on the flow passage side; and the pressurizing portion has a convex configuration of a size which allows insertion thereof into the concave configuration.
(33) A flow rate regulator as described in any one of items (28) to (31), wherein it has a scale for indicating a displaced amount of the pressurizing portion.
(34) A liquid medication set equipped with a flow rate regulator as described in any one of items (28) to (33).
(35) A flow rate switcher comprising of:
   a support body having an engagement portion; an operating member which is engaged such that it can be turned using the engagement portion as a fulcrum; a cylindrical body situated between the support body and the operating member, with the outer periphery partially supported by the support body, and which has an inlet and an outlet for a fluid; and a flow passage bottom member which is formed of a hard material and is in close contact with and in part closely fixed to the support body side of an interior of the cylindrical body forming a flow passage for the fluid; wherein:
      the operating portion has a first pressurizing portion and a second pressurizing portion to pressurize the flow passage side outer peripheral portion of the cylindrical body; the flow passage bottom member has a control groove extending in an axial direction of the cylindrical body; and in the vicinity of a boundary between the cylindrical body and the flow passage bottom member, a section configuration of both side end portions of the flow passage bottom member exhibits a continuous, gentle roundness; and
      a center portion thereof exhibits a concave configuration.
(36) The flow rate switcher as described in the item (35), wherein:
   the first pressurizing portion is arranged in a position where, at the time of pressurizing, it pressurizes the cylindrical member in cooperation with the flow passage bottom member; and the, second pressurizing portion is arranged in a position where, at the time of pressurizing, it pressurizes the cylindrical member in cooperation with the support member.
(37) The flow rate switcher as described in the item (35) or (36), wherein:
   the first pressurizing portion has a convex section configuration of a size which allows insertion thereof into the concave configuration; and a radius R of the continuous, gentle roundness of the section configuration of the both side end portions of the flow passage control member, and a thickness D of the cylindrical body are in the relationship: R ≥ D/10.
(38) The flow rate switcher as described in any one of items (35) to (37), wherein a circumferential length of an interior of the cylindrical body is set to be smaller than a peripheral length of the section of the flow passage bottom member.
(39) The flow rate switcher as described in any one of items (35) to (38), wherein:
   the cylindrical body has a deform portion in which a pressurized site deforms at the time of pressurization; the deformed deform portion and the flow passage bottom member are in close contact with each other excluding the control groove; the deform portion is constantly under a tensile strength in the inner side of the section taken perpendicularly to the axial direction of the cylindrical body; in a non-pressurized state, the section configuration of the deform portion as taken perpendicularly to the axial direction of the cylindrical body is linear; and at least a surface where the interior of the cylindrical body and the flow passage bottom member comes into contact with each other, in the vicinity of a flow passage formed by the deform portion and the flow passage bottom member, is a close contact portion that is not adhesion fixed.
(40) The flow rate switcher as described in any one of items (35) to (39), in which a numerical value indicative of a flow rate is described on the operating member.
(41) The flow rate switcher as described in any one of items (35) to (40), wherein the cylindrical body is composed of one of the followings, a liquid transfusion tube, a blood transfusion tube, or a nutritional transfusion tube.

The invention is defined by item (35), corresponding to claim 1. A device according to the preamble of claim 1 is disclosed in document DE 197 12 908.

### BRIEF DESCRIPTION OF THE DRAWINGS

Fig. 1 is a perspective view of a flow rate controller;
Fig. 2 is a perspective view of a flow passage control member integrally equipped with a connecting member;
Fig. 3 is a sectional view taken along the line A-A of Fig.1;
Fig. 4 is a sectional view taken along the line B-B of Fig.1;
Fig. 5 is a sectional view taken along the line C-C of Fig.1;
Fig. 6 is a sectional view when the flow rate of the flow rate controller is set to zero;
Fig. 7 is a sectional view when the flow rate of the flow rate controller is set to maximum;
Fig. 8 is a perspective view of a second flow rate controller;
Fig. 9 is a sectional view of a third flow rate controller;
Fig. 10 is a sectional view of a fourth flow rate controller;
Fig. 11 is a sectional view of another flow rate controller which differs from Fig.10 in arrangement of interception part and control groove;
Fig. 12 is a sectional view of a conventional flow rate controller;
Fig. 13 is a sectional view of a flow passage control member;
Fig. 14 is a sectional view of a further flow passage control member;
Fig. 15 is a perspective view of a flow rate regulator;
Fig. 16 is a perspective view of a flow passage bottom member integrally equipped with a connecting member composing a flow rate regulator;
Fig. 17 is a longitudinal sectional view showing an operation of the flow rate regulator. Fig. 17A shows a state where a flow rate is adjusted and Fig. 17B shows a state where the flow rate is zero;
Fig. 18 is a cross-sectional view showing an operation of an operating member. Fig. 18A shows the state where the flow rate is adjusted, and Fig. 18B shows a state where an infusion liquid is flushed;
Fig. 19 is a sectional view taken along the line V-V of Fig. 17B, in which an operating member is not shown;
Fig. 20 is a perspective view of a further flow rate regulator;
Fig. 21 is a longitudinal sectional view showing an operation of the latter flow rate regulator. Fig. 21A shows a state where a flow rate is at a predetermined level, and Fig. 21B shows a state where the flow rate is zero;
Fig. 22 is a cross-sectional view showing the operation of an operating member. Fig. 22A shows the state where the flow rate is at a predetermined level, and Fig. 22B shows a state where an infusion liquid is flushed;
Fig. 23 is a perspective view of another flow rate regulator;
Fig. 24 is a perspective view of a pressure-closing slider;
Fig. 25 is a cross-sectional view showing another structural example of a soft member;
Fig. 26 is a perspective view of a flow rate switcher according to the present invention;
Fig. 27 is a sectional view taken along the line A-A of Fig. 26, and shows a state where an infusion liquid is flushed;
Fig. 28 is a sectional view taken along the line A-A of Fig. 26, and shows a state where a flow rate is zero;
Fig. 29 is a sectional view taken along the line A-A of Fig. 26, and shows a state where the flow rate is adjusted; and
Fig. 30 is a sectional view of a cylindrical body composing the flow rate switcher according to the present invention, which is taken along the line B-B of Fig. 26, and shows the cylindrical body and a flow passage bottom member.

### BEST MODE FOR CARRYING OUT THE INVENTION

Embodiments of the present invention will now be described with reference to the accompanying drawings, which should not be construed restrictively.

The described flow rate adjusters can flow rate adjusters can be incorporated into a medical instrument which requires fluid flow rate adjustment, such as a fluid infusion set, a blood transfusion set, a nutritional transfusion set, a pressure monitoring line, an oxygenator circuit and a dialysis circuit.

It should be noted for the sake of convenience that the term axial direction indicates the direction in which fluid flows, and that the term direction perpendicular to the axial direction indicates a direction orthogonal to the axial direction.

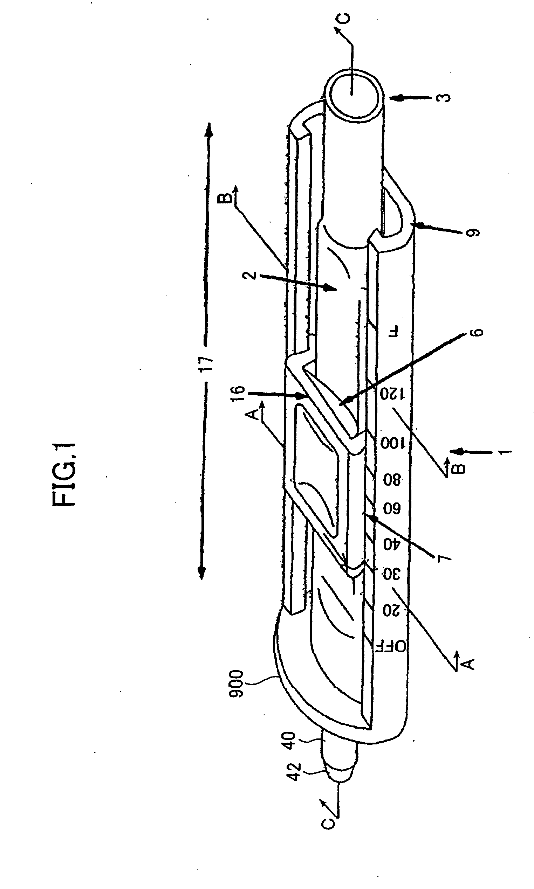

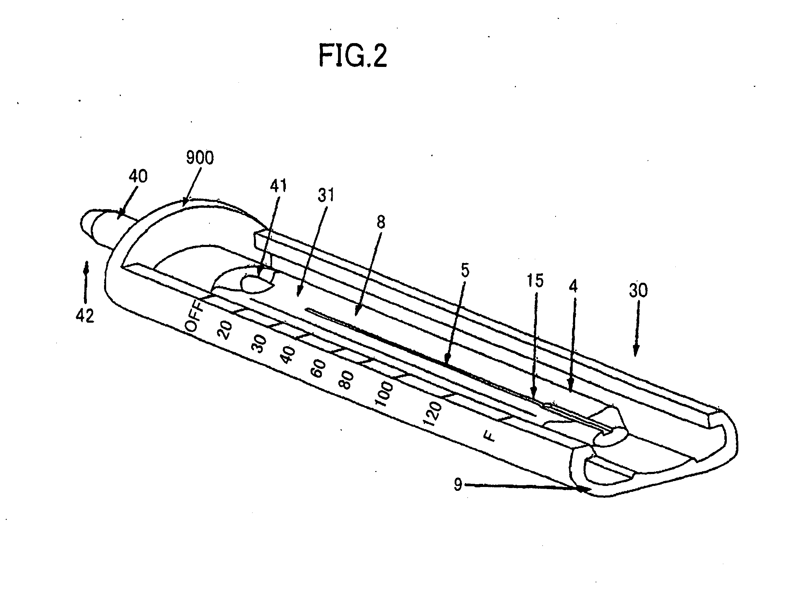

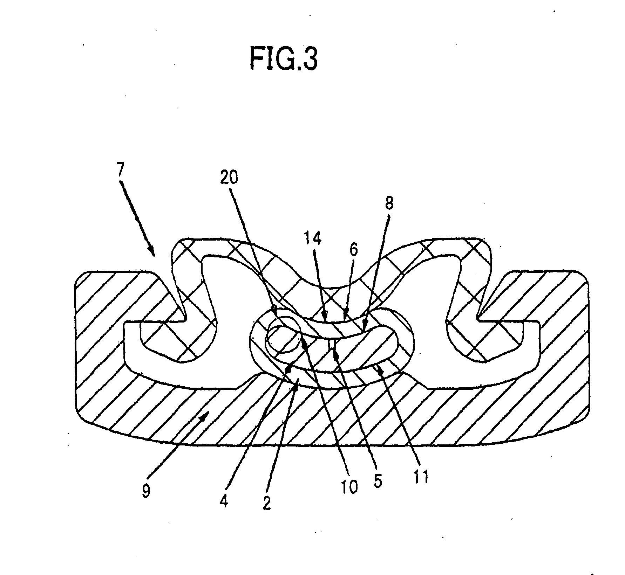

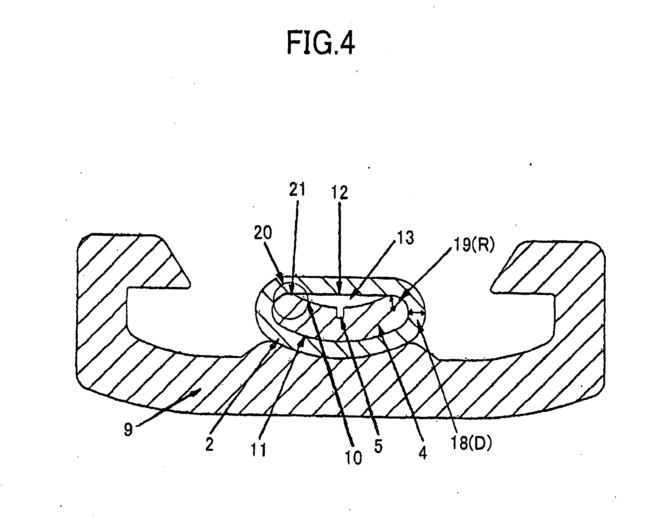

Fig. 1 is a perspective view of a flow rate controller; Fig. 2 is a perspective view of a flow passage control member integrally equipped with a connecting member; Fig. 3 is a sectional view taken along the line A-A of Fig. 1; and Fig. 4 is a sectional view taken along the line B-B of Fig.1.

There is described a flow rate controller 1 comprising of: a support body 9; an operating member 7 slidably engaged with the support body 9; a cylindrical body 2, outer periphery of which is partially supported by the support body 9 and has an inlet and an outlet for a fluid; and a flow passage control member 4 formed of a hard material and in close contact with and in part closely fixed to the support body side of the cylindrical body 2 forming a flow passage for the fluid. A fluid flows in from upstream end of flow passage control member 4, which will be explained later, and flows out of downstream end 3 of cylindrical body 2, after flow rate is controlled while passing through passage 13 between cylindrical body 2 and flow passage control member 4, or control groove 15.

As shown in Fig.1 and Fig.2, the operating member 7 of this flow rate controller 1 has a pressurizing portion 6 for pressurizing the flow passage side outer peripheral portion of the cylindrical body 2, and the cylindrical body 2 or the flow passage control member 4 has a control groove 15 extending in the axial direction 17 of the cylindrical body 2. As shown in Fig.3, the sectional form of both side end portions of the flow passage control member 4, in the vicinity of the boundary 20 between the cylindrical body 2 and the flow passage control member 4, wherein they exhibit a continuous, gentle roundness.

The flow rate controller 1 is equipped with the cylindrical body 2 formed of a soft material and is made to be flexible. A downstream end 3 of the cylindrical body 2 is connected in a liquid-tight manner to a downstream line (not shown) of a medical instrument such as a fluid infusion set, a blood transfusion set, a nutritional transfusion set. A downstream line of a medical instrument is connected to a patient. Further, cylindrical body 2 itself may be a tube composing a downstream line. Furthermore, the flow rate controller 1 is equipped with the operating member 7 having the pressurizing portion 6, and the support body 9.

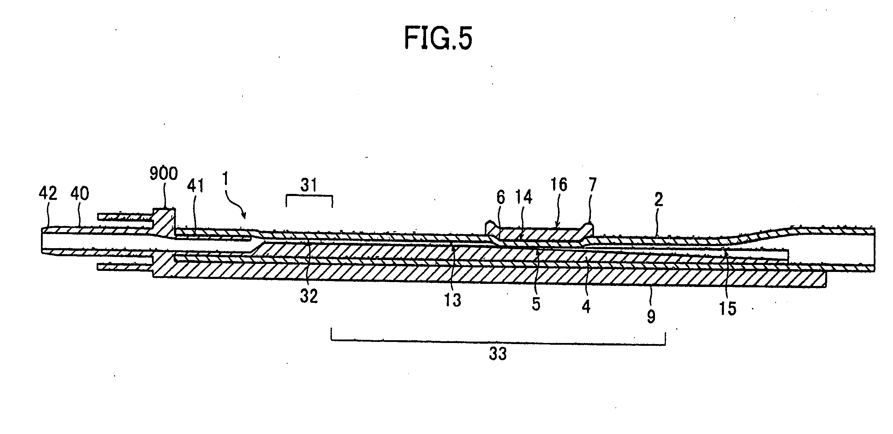

As shown in Fig.5, the flow passage control member 4, formed of a hard material extending in axial direction, is arranged inside the cylindrical body 2. The upstream end of flow passage control member 4 is made of a hard material and is integrally molded with a hollow tube like connecting member 40 protruding to both upstream and downstream side against upstream side end 900 of the support body 9. Upstream end of the cylindrical body 2 is fixed liquid-tightly to outer peripheral portion of connection path 41 protruding to downstream side of the connecting member 40. The cylindrical body 2 and the flow passage control member 4 arranged inside is closely fixed, by fixing a part of the inner surface of the former and a part of the outer surface of the latter, so as not to allow any liquid flow. As shown in Fig.4 and Fig.5, the flow passage forming surface (flow passage surface) 8, a part of outer peripheral portion of passage control member 4 not closely fixed to inner surface of the cylindrical body 2, forms a flow passage 13 for liquid to flow between inner surfaces of facing cylindrical body 2. Flow passage forming surface (flow passage surface) 8 is provided with flow rate adjusting passage 5 having control groove 15 with its sectional area gradually varying along the axial direction 17 to control the flow rate of the fluid. Control groove can be provided on flow passage surface of the cylindrical body, though not shown.

As shown in Fig.3 and Fig.4, the flow passage forming surface (flow passage surface) 8 that forms the flow passage has a form of an arcuate concave configuration exhibiting a gentle curve except for the part where the control groove 15 is formed. Further, the side end portions of the flow passage forming surface (flow passage surface) 8 (vicinity of the boundaries 20 between the flow passage control member 4 and the cylindrical body 2 when the pressurizing portion 6 is not acting) are also composed of gently rounded curved surfaces 10. The curvature of the gently rounded curved surfaces related here have a radius not less than 0.05 mm. The flow passage forming surface (flow passage surface) 8 may be partially formed in a linear configuration as long as the side end portions thereof (vicinity of the boundaries 20 between the flow passage control member 4 and the cylindrical body 2 when the pressurizing portion 6 is not acting) are formed as gently rounded curved surfaces. In other words, the flow passage forming surface (flow passage surface) 8, excluding control groove 15, may not entirely be formed in an arcuate concave configuration exhibiting a gentle curve, but may partially be formed in a flat plane surface. Further, it is also possible to adopt a structure in which this flat plane surface includes the adjustment flow passage. Furthermore, the surface having an arcuate concave configuration is not limited to a surface with single arcuate curve as shown in the drawing, but it is also possible to have a surface with wave-like continuous gently rounded curve.

As shown in Fig.3, the flow passage control member 4 has a concave section configuration to the axial direction of the cylindrical body 2 on the flow passage forming surface (flow passage surface) 8 side, and the pressurizing portion 6 has a convex section configuration of a size which allow insertion into the concave. A gently rounded configuration is possible if the radius 19 (R) of the section configuration of the side end portions of the flow passage control member 4, as shown in Fig.4, and the thickness 18 (D) of the cylindrical body 2 satisfies the following relationship: R ≥ D/10. If the radius R and the thickness D have the following relationship: R < D/10, the side end portions of the cylindrical body 2 can not be brought into close contact with the side end portions of the flow passage control member 4 when the pressurizing portion 6 pressurizes the cylindrical body 2, and a gap is generated between the flow passage control member 4 and the cylindrical body 2.

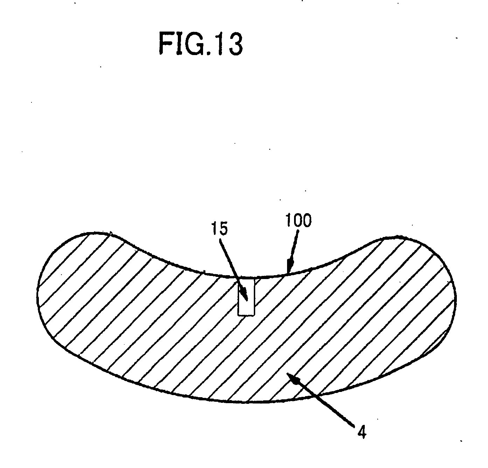

Further, when the flow passage control member 4 is incorporated into the interior of the cylindrical body 2, it is preferable that the inner peripheral length of the cylindrical body 2 be smaller than the section peripheral length 100 of the flow passage control member 4, as shown in Fig.13, excluding the control groove 15. With this arrangement, as will be explained in detail below, no gap is generated between the flow passage control member 4 and the cylindrical body 2 when the pressurizing portion 6 pressurizes the cylindrical body 2.

Assuming that the inner peripheral length of the cylindrical body 2 is L, and that the section peripheral length 100 of the flow passage control member 4 excluding the control groove 15 is M, it is more preferable to have following relationship: 0.6 < L/M <1. It is because if L/M is not less than 1, the cylindrical body 2 is loosened, and a gap is likely to be generated between the flow passage control member 4 and the cylindrical body 2 when the pressurizing portion 6 pressurizes the cylindrical body 2, and if L/M is not more than 0.6, the frictional resistance will increase as the pressurizing portion 6 pressurizes the cylindrical body 2 and lead to a poor operability, and lead to poor condition of unstable flow rate due to deformation caused by a great load applied to the flow passage control member and the cylindrical body.

Further, assuming that the inner peripheral length of the cylindrical body 2 is L, and that the section peripheral length 100 of the flow passage control member 4 excluding the control groove 15 is M, it is more preferable to have the following relationship: 0.9 < L/M < 1. With this arrangement, the frictional resistance is further reduced to improve the operability when the pressurizing portion 6 is moved, and further, elastic power of the cylindrical body 2 will be maintained for a long period of time.

As stated above, it is preferable to set the section peripheral length 100 of the flow passage control member 4, excluding the control groove 15, larger than the inner peripheral length of the cylindrical body 2. With this arrangement, a generation of a gap between the deformable portion 12 (deform portion) and the corresponding portion in the vicinity of the gently rounded curved surface 10 is further prevented when it is pressurized by the pressurizing portion 6. Further, it is preferable that the inner sectional surface of the deformable portion 12 (deform portion) is exposed to a tensile strength, in a direction to reduce the peripheral length. This is made possible by selecting such a material for the cylindrical body 2, as maintains a tensile strength even if heated for a sterilization and such, and resists against stress relaxation with a passage of time.

When a material relatively subject to stress relaxation is selected for the cylindrical body 2, as shown in Fig.4, it is possible to make the deformable portion 12 (deform portion) substantially linear. With this arrangement, further stabilization is achieved, and no gap other than the flow passage 13 is generated between the cylindrical body 2 and the flow passage control member 4 during both the pressurizing and non-pressurizing periods. As a result, it is possible to eliminate liquid retaining portions, and to bleed off air completely at the time of priming.

As mentioned above, the upstream end of passage control member 4 is integrally molded with the connecting member 40 protruding to both upstream and downstream side against upstream side end 900 of the support body 9 (see Fig.5). The connection path 41 protruding to downstream side of the connecting member 40 is connected to the fluid flow passage 13 and the adjustment flow passage 5. Although not shown, the portion 42 protruding to upstream side of the connecting member 40, on the other hand, is connected liquid-tightly to the upstream line of a medical instrument such as liquid infusion bag, blood transfusion bag, and nutritional transfusion bag. Further, although not shown, the upstream line consists of, for example, a liquid infusion bag, blood transfusion bag, nutrition bag, infuser pump, etc., and an upstream tube. It may also include a drip infusion cylinder.

The downstream end 3, at the downstream end of the cylindrical body 2 of the fluid flow passage 13 and the adjustment flow passage 5, is connected, through connecting member, to downstream line of medical instrument such as, a liquid infusion bag, a blood transfusion bag, a nutrition bag, and infuser pump, and the liquid is transmitted to a patient through a downstream tube of medical instrument. Further, the cylindrical body 2 and the tube that compose downstream line of medical instrument, such as a liquid infusion set, blood transfusion set, and nutrition set, may be integrally molded to reduce the number of parts and thus reduce the cost.

In the flow passage forming surface (flow passage surface) 8 of the flow passage control member 4, the control groove 15 constituting the adjustment flow passage 5 is formed extending in the axial direction of the flow passage control member 4, and in the case of Fig.5, the sectional area of the control groove 15 is gradually increasing from zero in the axial direction toward the downstream side by increasing the depth of the adjustment flow passage 5 in the above mentioned direction. In order to change the sectional area of the control groove 15, it is also possible to vary the width or both the width and the depth of the control groove 15. Further, on the contrary, it is also possible for the sectional area of the control groove 15 to gradually increase toward the upstream side.

When an infusion liquid (liquid medicine), for example, such as lactated Ringer's solution, physiological saline, is transfused from a transfusion bag to a vein by, for example, the general gravitation system, it is preferable that the sectional area of the control groove 15 be approximately 0 to 0.5 mm². However, the preferred range of the sectional area of the control groove 15 varies in accordance with the section configuration of the control groove 15, the viscosity and other nature of the fluid, the flow rate, the means for imparting pressure to the fluid (e.g. head drop system, a system using a pressurizing means, such as a balloon), etc. Thus, it goes without saying that the range of the cross sectional area of the control groove 15 is not restricted to the above mentioned range.

Further, while in the example shown in Fig.13, the section configuration of the control groove 15 is rectangular, it may also be a U-shaped or a semi-spherical configuration and it is preferable for the control groove to have a concave form. Further, while the control groove 15 extends linearly in the axial direction of the flow passage control member 4 in this example, it may also extend in a wave-like line. The number of control grooves 15 may be one or two or more. Forming a single groove is advantageous in that it helps to restrain variation in flow rate due to air blocking.

As shown in Fig.1, the outer periphery of the cylindrical body 2 is provided with the support body 9 and the operating member 7 having the pressurizing portion 6, and the operating member 7 is movable along the axial direction of the flow passage control member 4. The operating member 7 has an operating portion 16 to move in the axial direction of the flow passage control member 4, and the pressurizing portion 6 to pressurize the cylindrical body 2. Further, the support portion 9 may be composed as support body 9 by integrally molded with the operating member 7. The support portion 9 or the support body 9 will cooperate with the flow passage control member 4 to support the cylindrical body 2 so as to surround it.

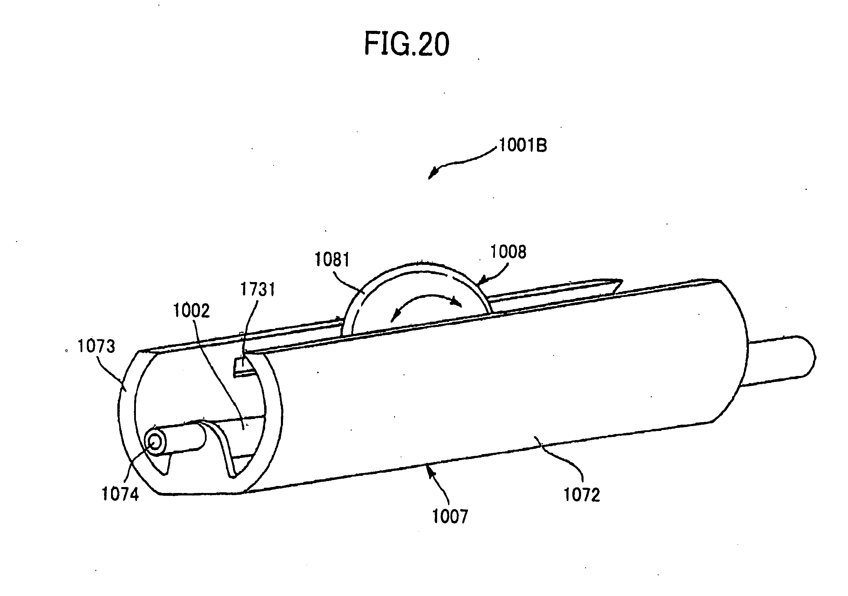

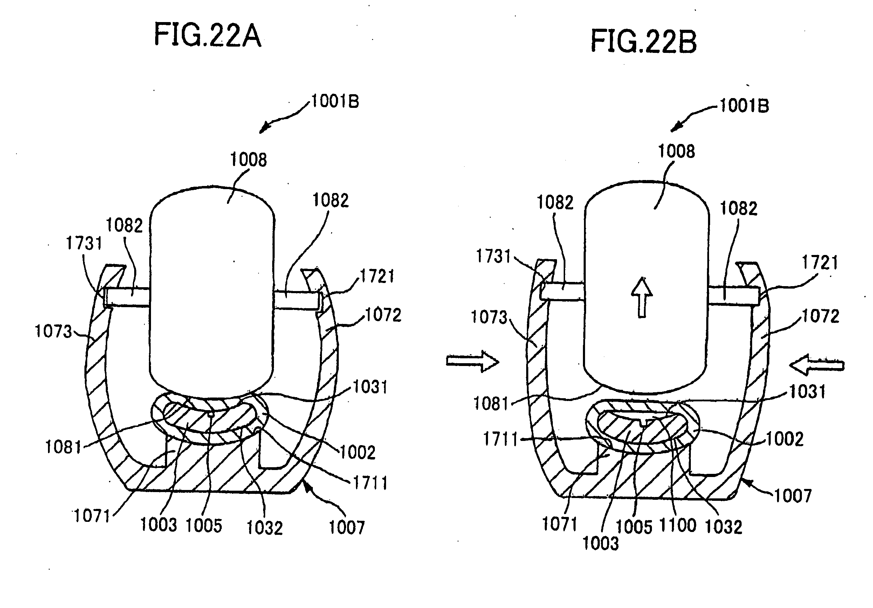

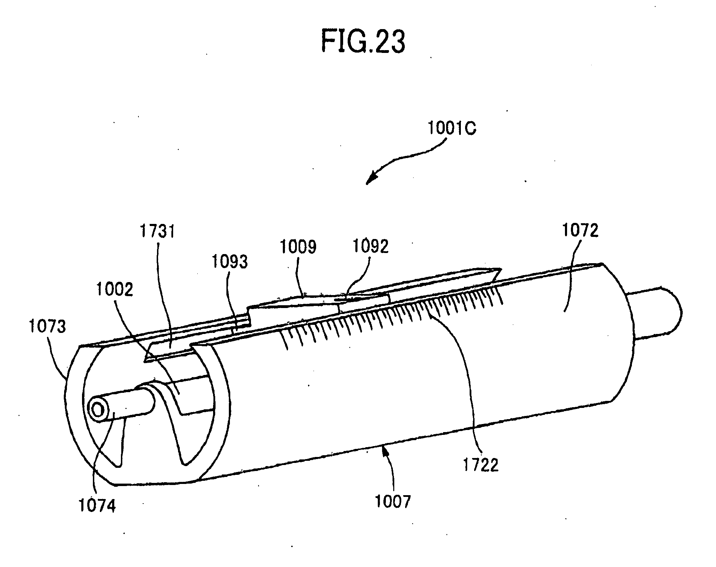

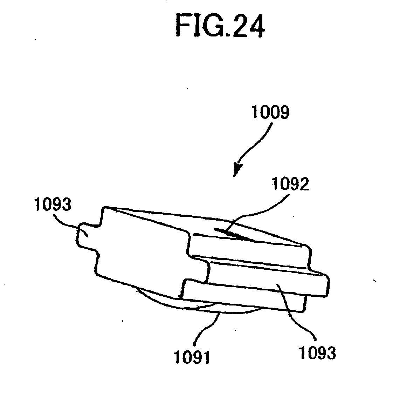

As long as the above-described basic construction is maintained, there is no particular restriction regarding its mechanism. The pressurizing portion 6 of the operating member 7 may also be a slider adapted to slide while pressurizing the cylindrical body as will be explained later in connection with Figs.23 and 24, or a roller adapted to rotate while pressurizing the cylindrical body 2 as will be explained later in connection with Figs.20 and 22.

In Fig. 1, the flow rate controller 1 can adjust flow rate by moving the support portion 9 (or support body 9 integrally molded with the operating member 7) along the axial direction 17 of the cylindrical body 2. It is preferable for the pressurizing surface 14 of the pressurizing portion 6 to be of a configuration in conformity with the flow passage surface 8 of the flow passage control member 4, that is, a convex configuration exhibiting a gentle curve. Further, it is preferable that the length of the pressurizing surface 14 in the axial direction is smaller than that of the control groove 15 in the axial direction. When the length of the pressurizing surface 14 in the axial direction is smaller than that of the control groove 15 in the axial direction, flow rate of flow rate controller 1 can be adjusted preferably by moving pressurizing surface 14 along the axial direction while pressurizing cylindrical body 2 by pressurizing surface 14.

As shown in Fig.4, the inner surface of the cylindrical body 2 includes a deformable portion 12 (deform portion) that can be deformed by being pressurized by the pressurizing portion 6, a part of the outer surface of the flow passage control member 4 as stated above, and a fixed portion 11 which is closely fixed so as not to allow passage of fluid.

When the pressurizing portion 6 is not operating, the near the boundary portions 20 of the flow passage control member 4 and the cylindrical body 2 may have close contact portions 21, where the portions of the cylindrical body 2 are, though not fixed, in close contact with the flow passage control member 4. In this state, the fixed portion 11 is in close contact with the close contact portions 21. With the provision of the close contact portions 21, distortion is mitigated, and the level difference on the surface forming the flow passage is eliminated, when the pressurizing portion 6 pressurizes the cylindrical body 2. Thus, near the boundary portions 20, there is no gap between the cylindrical body 2 and the flow passage control member 4. As a result, it is possible to substantially block the flow passage 13 still more easily.

When the deformable portion 12 (deform portion) is pressurized by the pressurizing portion 6, it exhibits a configuration which coincides with the section configuration of the concave flow passage forming surface (flow passage surface) 8 formed by a gentle curve, and the section configuration of the gently rounded curved surface 10 on both side end portions thereof. As stated above, it is also preferable for the section configuration of the pressurizing portion 6 to be similar to the above-described one.

With this arrangement, the deformable portion 12 (deform portion) will have no gap between positions corresponding to the gently rounded curved surfaces 10, when it is pressurized by the pressurizing portion 6. At this time, as shown in Fig.3, the flow passage 13 for the fluid is completely closed excluding the adjustment flow passage 5 (control groove 15). Thus, only the adjustment flow passage 5 (control groove 15) constitutes the flow passage for the fluid, thereby achieving coincidence with the flow rate set by the adjustment flow passage 5 (control groove 15).

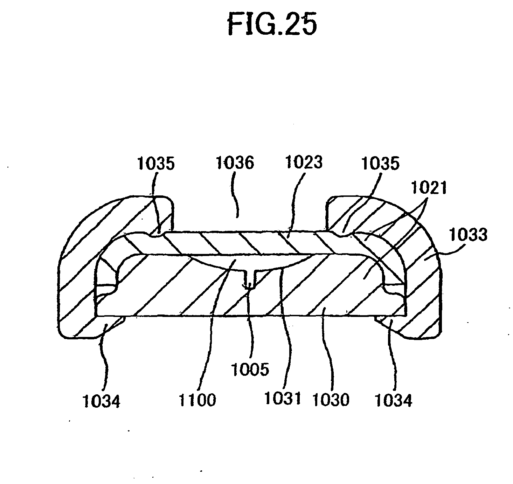

Further, the cylindrical body 2 and flow passage control member 4 is not restricted to the above mentioned form as long as it exhibits a configuration which coincides with the section configuration of the concave flow passage forming surface (flow passage surface). 8 of flow passage control member 4, and the gently rounded curved surface 10 on both side end portions thereof, when pressurized by the pressurizing portion 6. For example, instead of the cylinder shaped cylindrical body 2, cylindrical body may be consisting of an elastic deformable membrane-like soft material portion, and a flow passage control portion (flow passage bottom member) consisting of a hard material liquid-tightly connected to form a flow passage. Structure of the cylindrical body is omitted here, as it will be explained in detail referring to Fig.25. This cylindrical member can easily be produced by ultrasonic welding of the flow passage (formation) control portion (flow passage bottom member) consisting of a hard material and the membrane-like portion consisting of a soft material.

When the flow passage is thus formed liquid-tightly by the membrane-like portion and the flow passage control member (flow passage bottom member), it is preferable that the sectional length perpendicular to axial direction of the section of the deformable portion (a membrane-like portion that come into close contact with the flow passage surface of the section of the flow passage control portion (flow passage bottom member) when pressurized by the pressurizing portion) of the membrane-like portion to be such that, when pressurized by the pressurizing portion, the deformable portion (deform portion) expands so as to come into close contact with the flow passage surface of the section of the flow passage control portion (flow passage bottom member), excluding the control groove. Thus, it is preferable that the sectional length of the deformable portion of a membrane-like portion be smaller than the sectional length of the flow passage surface of the section of the flow passage control portion (flow passage bottom member) excluding the control groove. By thus setting the sectional length of the deformable portion of a membrane-like portion, no gap is generated as in the case of the above-described cylindrical body 2. Further, it is preferable that a tensile strength of not less than zero be applied to the inner section of the deformable portion, and when the pressurizing portion is not pressurizing, it is preferable that the deformable portion (deform portion) be linear.

With this arrangement, no gap is generated between the cylindrical body 2 or the deformable portion of a membrane-like portion of the cylindrical body, and the flow passage control member 4 or the flow passage control portion (flow passage bottom member) of the cylindrical body except for the adjustment flow passage, making it possible to eliminate a portion where liquid stagnates and to bleed air completely at the time of priming. It is preferable to execute a friction reducing process to at least the portion of the surface of the deformable portion 12 (deform portion) coming into contact with the pressurizing portion 6 corresponding to the axial length of the flow passage control member 4. Then the operation of moving the operating member 7 can be facilitated. The friction reducing process may, for example, be surface roughening, application of oil such as silicone, film coating with membrane such as PTFE, or multi-layer molding, but not particularly restricted as long as it helps to reduce friction between the pressurizing portion 6 and the deformable portion (deform portion) 12.

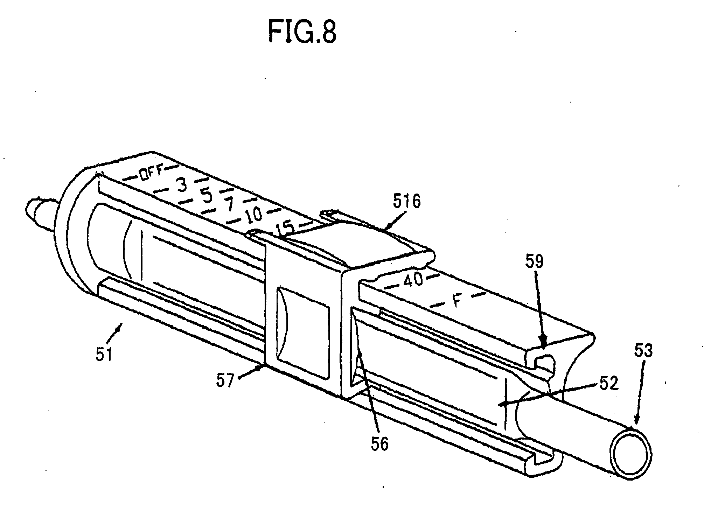

Fig.8 shows a perspective view of a flow rate controller 51.

The operating member 57 consists of the operating portion 516 to move the operating member 57 in axial direction of the support body 9, and the operating portion 516 is formed on different position in direction to pressurizing force caused by the pressurizing portion 56 on operating member 57 in relation to the support body 9. In case of the drawing, the operating portion 516 is engaged with the support body 59 at perpendicular position to one end of the pressurizing portion 56 at slidable position. A scale is also placed on the operating portion 516 plane. Position and structure of the operating portion 516 is satisfied as long as it is placed on the plane other than that of the pressurizing portion 56 on the operating member 57, and the pressurizing portion 56 can be moved easily when the operating portion 516 is pushed by a finger, and it can be a handle-like grip placed on the same plane as the operating portion 516 of the drawing.

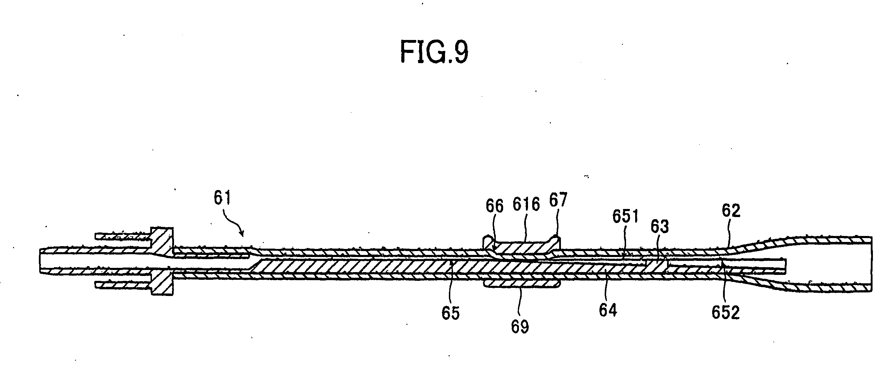

Fig.9 shows a perspective view of a flow rate controller 61. The flow rate controller 61 consists of the operating member 67, which is integrally molded in a cylindrical shape with the pressurizing portion 66, the operating portion 616 and the support body 69, and the flow passage control member 64 having intercepting portion 63. The intercepting portion 63 may be at any position within the control groove 65 and a plurality of it can be provided. It is needless to say that the section area of the downstream side control groove 652 and upstream side control groove 651 can be set arbitrarily. The structure of the operating member 67 is not referred here, as it will be explained in detail below.

The materials for the respective components of the flow rate controller 1 will be described. It is preferable for the material for the cylindrical body 2 to be an elastic one adapted to be elastically deformed within a certain range. Examples of the material include various rubber materials, such as silicone rubber, natural rubber, isoprene rubber, butadiene rubber, styrene-butadiene rubber, butyl rubber, and fluoro rubber; polyolefines, such as polybutadiene, EVA, soft polypropylene, and polyethylene; various thermoplastic resins, such as styrene-type elastomer, polyvinylchloride, and fluororesin; and mixtures including the above materials. It is preferable for the material to be transparent or semi-transparent so that the visibility of the interior of the cylindrical body may be secured and the presence of bubbles or the like may be ascertained.

When the various rubber materials are used for the cylindrical body 2, the flow passage control member 4 is inserted into the rubber tube with its inner periphery enlarged, so that a caulking force is applied to the flow passage control member and a lateral tensile strength is constantly applied to the deformable portion 12 (deform portion) which is deformed in the pressurizing portion shown in Fig. 4. As a result, even if the pressurization is continued for a long period of time, a stable pressurizing state is realized in which the cylindrical body undergoes no irreversible deformation that would allow fluid to flow in portions other than the control groove during pressurization. Further, in the case of a soft material, which is usually subjected to creep deformation when the control member is caused to slide vigorously or a fixed portion of the cylindrical body is kept pressurized for a long period of time, even when the device is used in such a manner as to cause permanent deformation which leads to the rattling of the slide control member at the time of sliding, flow rate control can be performed in a stable manner.

It is preferable that the material for the flow passage control member 4 be hard enough to form a stable adjustment flow passage 5. Examples of the material include high polymer materials, such as polycarbonate, polypropylene, high density polyethylene, polyamide, ABS resin, polyvinylchloride, polymethyl methacrylate, polyacetal, polysulfone, polyether sulfone, polyallylate, polyethylene terephthalate, polyethylene naphthalate, polyphenylene sulfide, polybutylene terephthalate, acrylonitrile-styrene resin, polyimide, polyamide imide, polymethyl pentene, PEEK, and fluoresin; and metal materials, such as titanium, and stainless steel. The same material can be used for the joint member integrally molded with the flow passage control member 4.

In particular, polypropylene, high density polyethylene, polyethylene naphthalate, polyphenylene sulfide, polybutylene terephthalate, polyether sulfone, polyamide imide, PEEK, etc. are superior in chemical resistance, and polycarbonate, polypropylene, ABS resin, polyacetal, etc. are superior in terms of balance among cost, resin fluidity at the time of molding, and heat resistance.

It is preferable that the material for the operating member 7 be the one appropriately hard enough to cause deformation of the cylindrical body 2. Examples of the material include: high polymer materials, such as polycarbonate, polyurethane, polypropylene, polyethylene, high density polyethylene, polyamide, ABS resin, polyvinylchloride, polymethyl matacrylate, polyacetal, polysulfone, polyether sulfone, polyallylate, polyethylene terephthalate, polyethylene naphthalate, polyphenylene sulfide, polybutylene terephthalate, acrylnitrile styrene resin, polyimide, polyamide imide, polymethyl pentene, PEEK, and fluororesin; and metal materials, such as titanium, and stainless steel.

### (Embodiment 1)

Next, the construction of a flow rate controller will be described. As the cylindrical body 2, a soft tube having an inner peripheral length of 6.6 mm and a thickness 18 (D)(see fig.4) of 0.6 mm and consisting of polybutadiene (manufactured by JSR, Co.) was prepared by extrusion molding. The flow passage control member 4 was prepared as the same component as the support body 9 by using polypropylene (manufactured by Nippon Polychem, Co.). As shown in Fig.5, the flow passage surface 8 of the flow passage control member 4 has an interception region 31 and a flow rate control section 33, where the control groove 15 is provided. The control groove 15 extends 32 mm from the end portion of the flow rate control section 33, and its depth and width are adjusted so that its sectional area varies toward the upstream side from 0.064mm² to 0.016 mm², thereby preparing the adjustment flow passage 5.

The radius 19 (R) of the continuous gently rounded curved surfaces in the vicinity of the boundary portions 20 at the side end portions of the flow passage control member 4 shown in Fig.4 is 0.5 mm. The radius of the concave of the flow passage control member 4 excluding the control groove 15 is 2 mm, the width of the flow passage control member 4 is 3.5 mm, the length thereof is 60 mm, the section peripheral length 100 excluding the control groove 15 is 8.4 mm, and as shown in Fig. 4, excluding the control groove 15, the section outer periphery of the flow passage control member 4 constitutes an edgeless smooth surface. As shown in Fig. 1, a scale is provided on a side surface of the support body 9.

Assembly is conducted by inserting an end portion of the soft tube (cylindrical body 2) from the downstream end 30 of the flow passage control member 4, the inner peripheral length of the cylindrical body 2 being 8.3 mm and assuming that the inner peripheral length of the cylindrical body 2 is L and that the section peripheral length 100 of the flow passage control member 4 is M, the ratio resulted in L/M = 0.99. As shown in Fig.2, the end portion of the downstream end 30 of the flow passage control member 4 is tapered toward the downstream side, facilitating the insertion of the soft tube (cylindrical body 2). When the flow passage control member 4 is inserted into the interior of the cylindrical body 2 upon assembling, the section peripheral length 100 of the flow passage control member 4 excluding the control groove 15 is larger than the inner peripheral length of the soft tube (cylindrical body 2), so that tensile strength is applied to the deformable portion 12 (deform portion) of the soft tube (cylindrical body 2) corresponding to the flow passage surface 8, and, as shown in Fig. 4, the deformable portion 12 (deform portion) is linear.

The operating member 7 is prepared by using high density polyethylene (manufactured by Nippon Polychem, Co.). After fitting the soft tube onto the flow passage control member 4, engagement is effected such that the sectional view, as shown in the Fig.3 and Fig.4, shows that the cylindrical body 2 is held between it and the support body 9. As can be seen from Fig.1, the operating member 7 is capable of moving by the length corresponding to the scale provided on the support body 9. The pressurizing portion 6 of the operating member 7 has a rounded convex configuration with the radius of the roundness 1.56 mm and the pressurizing portion can be inserted into the concave of the flow passage control member 4. A minute amount of silicone oil is applied to the pressurizing surface 14 for lubrication. With the application of the silicone oil, the operating member 7 can be moved more easily with one hand.

Next, the way the flow rate controller is used will be described. The flow rate controller 1 was incorporated into a liquid infusion set, and a transfusion bag containing physiological saline as transfusion was connected to a supply needle so that the total length of the system was 130 cm, with physiological saline flowing with a head drop of 70 cm. When adjusting the flow rate of the physiological saline in this state, the operating member 7 is moved in the axial direction of the flow passage control member 4, as shown in Fig.5, and a predetermined portion of the cylindrical body 2 is pressurized by the pressurizing portion 6 of the operating member 7. Whereby a predetermined portion of the deformable portion 12 (deform portion) of the cylindrical body 2 is deformed to come into close contact with the flow passage surface 8 of the flow passage control member 4.

At this time, due to the continuous gently rounded configuration of the end portions 10 of the flow passage control member 4, the section configuration of the flow passage control member 4 in the axial direction of the flow passage control member 4 does not involve generation of a gap when the restoring force is generated by pressurizing the cylindrical body 2, and the section configurations of the close contact surface and the close contact fixing surface of the flow passage control member 4 and the cylindrical body 2 coincide with each other excluding the control groove 15. Thus, the flow passage 13 is completely closed excluding the adjustment flow passage 5, whereby the portion of the control groove 15 covered with the cylindrical body 2, that is, the cross-sectional area of the adjustment flow passage 5 is varied to vary the duct resistance of the adjustment flow passage 5, making it possible to adjust the flow rate of the transfusion.

Further, it is preferable that a tensile strength of not less than zero be constantly applied to the inner section of the deformable portion 12 (deform portion), and when the pressurizing portion 6 is not pressurizing, it is preferable that the deformable portion be linear as shown in Fig.4. With this arrangement, the deformable portion 12 (deform portion), when pressurized by the pressurizing portion 6, is less likely to involve generation of a gap between it and the portions corresponding to the portions in the vicinity of the gently rounded curved surfaces 10.

Here, it is also possible to effect a non-linear setting of the variation amount of the sectional area of the control groove 15 and the displacement amount of the operating member 7 when adjusting the flow rate. Further, it is also possible to effect setting such that adjustment for low flow rate is more accurate than that for high flow rate or, conversely, adjustment for high flow rate is more accurate than that for low flow rate.

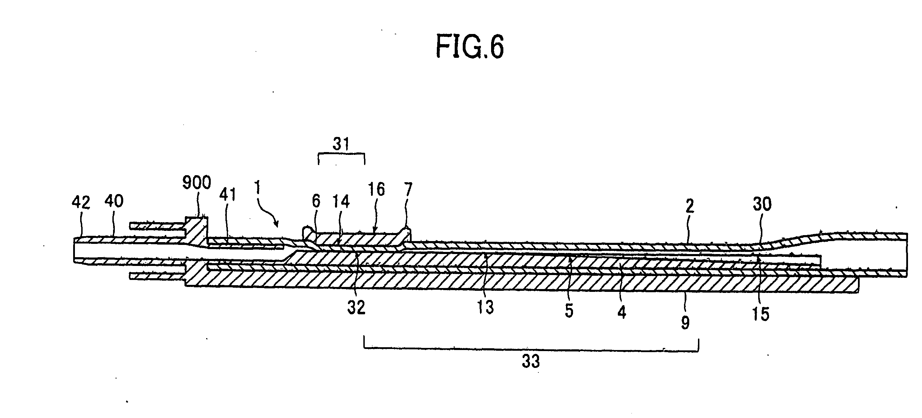

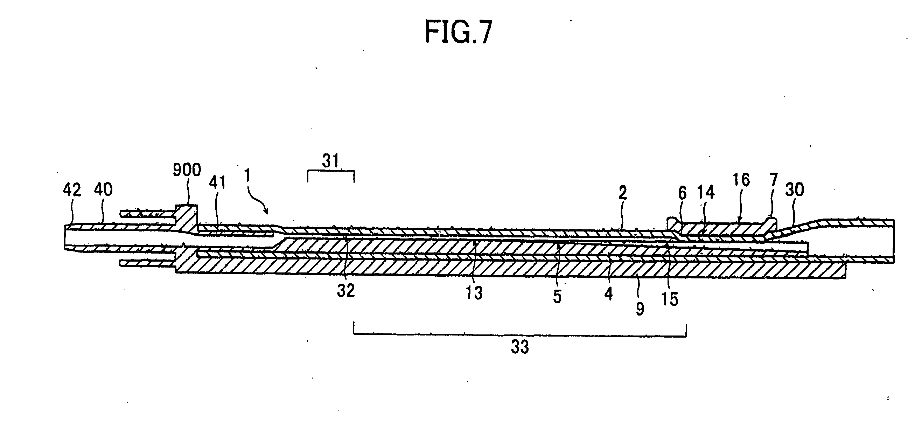

Next, when reducing the flow rate of the transfusion liquid to zero in the above-described state, the operator moves the operating portion 16 of the operating member 7 with a finger or the like toward the upstream side, as shown in Fig.6, and the portion of the cylindrical body 2 near the upstream end 32 is pressurized by the pressurizing portion 6 to bring deformable portion 12 of cylindrical body 2 into close contact with the interception region 31 at the upstream end 32 of flow passage control member 4. Whereby the flow passage 13 is closed, and since there is no adjustment flow passage in the interception region 31, the flow rate of the transfusion liquid is zero. When, as shown in Fig. 7, the operating member 7 is moved to the downstream end, the flow rate of the transfusion liquid becomes maximum.

As described above, when the flow rate of transfusion liquid is adjusted, no gap is generated between the flow passage control member 4 and the cylindrical body 2, and the passage is closed except for the adjustment flow passage 5, so that it is possible to maintain the flow rate adjusted by the control groove 15. Further, when the flow rate is adjusted to zero, the flow passage 13 is completely closed, so that no transfusion liquid flows.

While the flow rate may be set by counting the number of drips of the drip infusion cylinder, it is also possible to adjust and set the flow rate of the transfusion liquid by providing a predetermined scale on the support body 9. In that case, if the flow rate is temporarily made maximum (flashing) upon the transfusion, it can be easily restored to the same flow rate by means of the scale.

Thus, it is possible to easily set the flow rate to a desired level by means of a scale, and the re-setting after the flashing is also easy. When liquid was allowed to flow for six hours at a rate of 100 ml/hour, no difference in flow speed was ascertained between the start and end based on the count number of the drip infusion cylinder. Further, due to the scale, the set position for the operating member 7 could be easily confirmed.

Fig. 8 is a perspective view of a flow rate controller 51. Polypropylene (manufactured by Nippon Polychem, co.) is used for a support body 59, preparing a flow passage control member.

An operating member 57 is prepared by using a high density polyethylene (manufactured by Nippon Polychem, co.) and after fitting a soft tube onto the flow passage control member, engaged with the support body 59 so as to hold a cylindrical body 52 in between. A minute amount of silicone oil is applied to a pressurizing portion 56 for lubrication. In order that the frictional resistance of the pressurizing portion 56 may not be easily increased when force is applied to an operating portion 516 with a finger, the operating member 57 is prepared so that the operating portion 516 is perpendicular from one end portion of the pressurizing portion 56.

By thus reducing the contact area between the pressurizing portion 56 and the cylindrical body 52 during operation, it is possible to achieve a lighter operability and easily set flow rate. In order to achieve a lighter operability, the positional relationship is not limited to that in which the operating portion 516 is perpendicular from one end portion of the pressurizing portion 56, but can be achieved as long as the operating portion is positioned in a direction different from the direction in which the pressurizing force of the pressurizing portion is applied.

Next, the operation of a flow rate controller 61will be described. Fig. 9 is a sectional view of a flow rate controller 61. This flow rate controller 61 is composed of a cylindrical body 62, a flow passage control member 64, and an operating member 67. The operating member 67 is formed by using a high density polyethylene (manufactured by Nippon Polychem, co.), in which a pressurizing portion 66, an operating portion 616, and support portion 69 are integrally formed in a cylindrical configuration, and it is movable in the axial direction of the cylindrical body 62.

A control groove 65 is provided in a flow passage control member 64 of the flow rate controller 61. In the control groove 65, there is provided an intercepting portion 63 for intercepting fluid flow. This intercepting portion 63 divides the control groove 65 into an upstream side control groove 651 and a downstream side control groove 652. The upstream side control groove 651 has a length of 32 mm, and its depth and width are adjusted such that its sectional area varies toward the upstream side from 0.064 mm² to 0.016 mm². The sectional area of the downstream side control groove 652 is 0.64 mm², which is larger than that of the upstream side control groove 651, allowing a large amount of fluid to be conveyed therethrough.

The flow rate controller 61 was incorporated into a liquid infusion set, and a liquid infusion bag containing physiological saline was connected to a supply needle, with the total length of the system 130 cm, and the physiological saline flowing down with a head drop of 70 cm. When the physiological saline was flown in this state, it was possible to easily set the flow rate to a desired level at a position in the upstream side control groove 651 by means of a scale. When the pressurizing portion 66 of the operating member 67 was moved to the position of the intercepting portion 63, the flow rate of the fluid was zero. When the pressurizing portion 66 of the operating member 67 was further moved to the position of the downstream side control groove 652, a large amount of physiological saline flowed.

With the provision of the intercepting portion 63, it does not easily happen that an excessive amount of fluid is erroneously allowed to flow at the time of flow rate setting, making it possible to set flow rate with higher reliability.

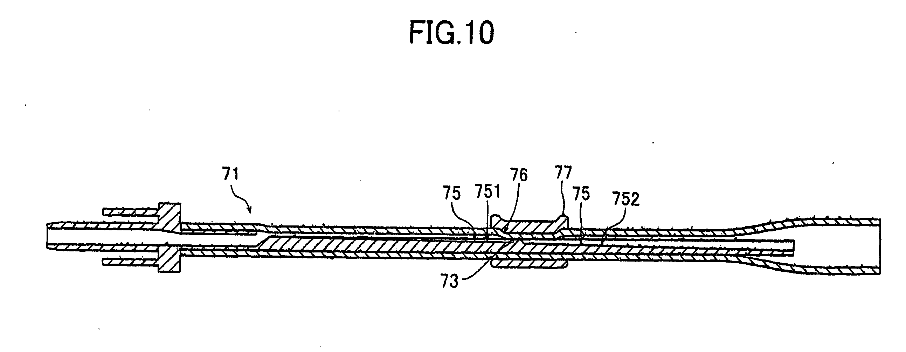

Next, the operation of a flow rate controller 71 will be described. Fig.10 is a sectional view of a flow rate controller 71. An intercepting portion 73 divides a control groove 75 into an upstream side control groove 751 and a downstream side control groove 752, and the sectional areas of the upstream side control groove 751 and the downstream side control groove 752 differs from the third embodiment. The upstream side control groove 751 has a length of 16 mm, and its depth and width are adjusted such that its sectional area varies from 0.043 mm² to 0.016 mm². The downstream side control groove 752 has a sectional area larger than that of the upstream side control groove 751, and has a length of 16 mm, and its depth and width are adjusted such that its sectional area varies from 0.074 mm² to 0.043 mm².

The flow rate controller 71 was incorporated into a liquid infusion set, and a liquid infusion bag containing physiological saline was connected to a supply needle, with the total length of the system 130 cm, and the physiological saline flowing down with a head drop of 70 cm. When the physiological saline was flown in this state, the flow rate could be easily set to a desired level at the position of the upstream side control groove 751 by means of a scale. When the pressurizing portion 76 of the operating member 77 was moved to the position of the intercepting portion 73, the flow rate of the fluid was zero. At the position of the downstream side control groove 752, a flow rate larger than that at the position of the upstream side control groove 751 could be easily controlled by means of the scale.

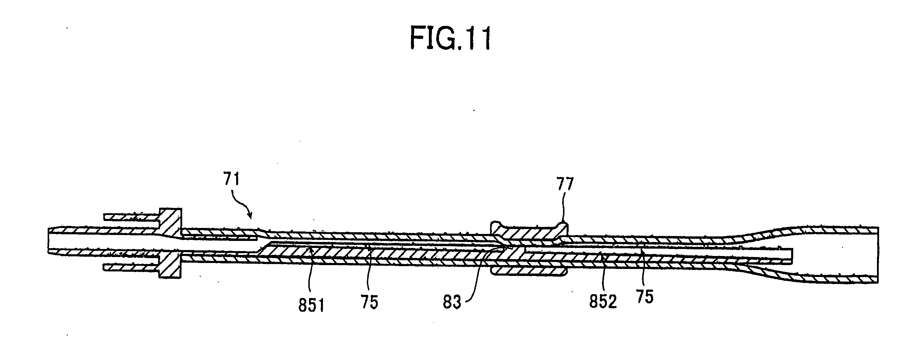

With the division of the control groove by the intercepting portion 73, setting can be easily conducted respectively for a case in which a minute amount of fluid is flown and for case in which a relatively large amount of fluid is flown. The intercepting portion 73 can be provided at an arbitrary position in the control groove 75 and can be provided in plurality. It goes without saying that the sectional areas of the downstream side control groove 752 and the upstream side control groove 751 can be arbitrarily set. Further, as shown in Fig.11, it is also possible to arrange control grooves 851 and 852 so as to increase the flow rate, using an intercepting portion 83 as an index point.

Next, a further flow rate controller will be described. Instead of the polybutadiene tube, an isoprene rubber tube having an inner peripheral length of 6.3 mm and a thickness of 0.62 mm is used, a liquid infusion set tube being connected to the end portion on the opposite side of the insertion end portion of the flow passage control member through the intermediation of a connector. The pressurizing portion has a rounded convex configuration, the radius of the roundness being 1.56 mm, and it can be inserted into a concave of the flow passage control member. A control device (not shown) was prepared, with a 12 micron polyethylene terephthalate membrane being placed between the cylindrical body and the operating member. The operating member can slide easily, and flow rate setting can be effected easily by means of a scale.

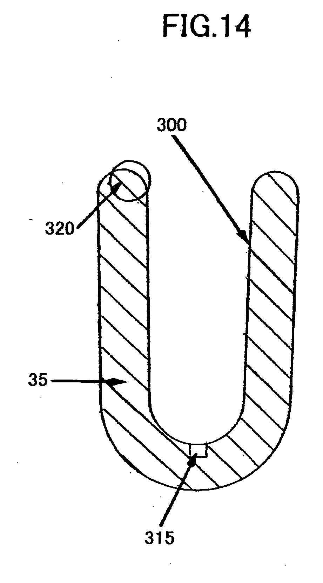

Fig.14 is a sectional view of a flow passage control member. A silicone rubber tube having an inner peripheral length of 20.0 mm and a thickness of 0.4 mm is used as the cylindrical body. The radius of a continuous, gently rounded near-boundary portion 320 at a side end portion of a flow passage control member 35 shown in Fig. 14 is 0.5 mm. The radius of the concave of the flow passage control member 35 excluding a control groove 315 is 1.0 mm, the width of the flow passage control member 35 is 4.2 mm, its length is 60 mm, the width of the section peripheral length 300 excluding the control groove is 30.4 mm, and, excluding the control groove 315, the section outer periphery of the flow passage control member 35 constitutes an edgeless smooth surface, as shown in Fig.14. Assuming that the inner peripheral length of the cylindrical body is L' and that the peripheral length of the section of the flow passage control member is M', L'/M' = 0.66. Further, although not shown, the pressurizing portion of the operating member has a convex configuration corresponding to the concave configuration of the flow passage control member. To ensure the surface slidability of the cylindrical body, a minute amount of silicone oil is applied thereto. The operating member can slide easily, and flow rate setting can be easily effected by means of a scale.

### (Comparative Example)

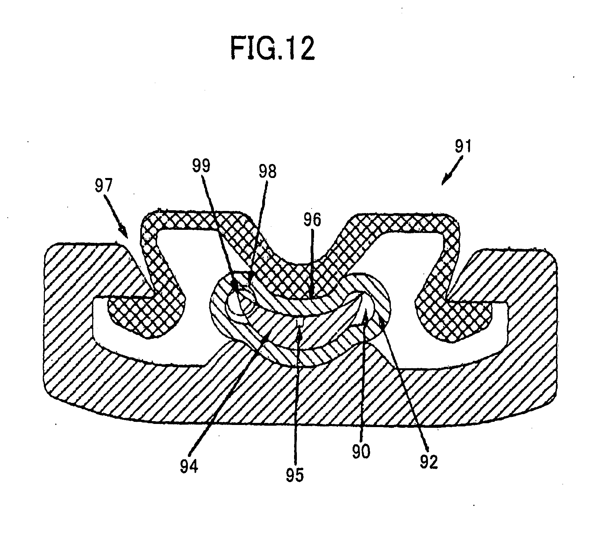

Fig. 12 is a sectional view of a conventional flow rate controller 91 in the axial direction thereof. As shown in Fig. 12, a pressurizing portion 96 of an operating member 97 pressurizes a cylindrical body 92 formed of a soft material. A tip end part 99 of an end portion 98 of a flow passage control member 94 formed of a hard material ends in a non-curved section configuration. In this case, the cylindrical body 92 pressurized by the pressurizing portion 96 undergoes elastic deformation and, due to its restoring force, it cannot be in conformity with the non-curved section configuration of the tip end part 99 of the end portion 98 of the flow passage control member 94, with the result that a gap 90 is formed. As a result, fluid flows out through the gap 90, and the flow rate set by the adjustment flow passage 95 is exceeded.

There are also described flow rate regulators. The flow rate regulators adjust the flow rate of a fluid such as an infusion liquid, transfusion blood, and transfusion nutrition, and are mainly incorporated into a liquid administering set such as a liquid infusion set, blood transfusion set, or nutritional transfusion set for liquid infusion, blood transfusion, or nutritional transfusion, respectively, before use.

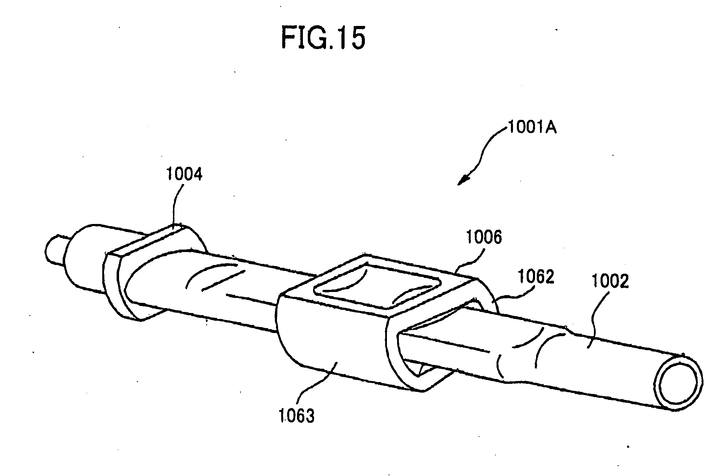

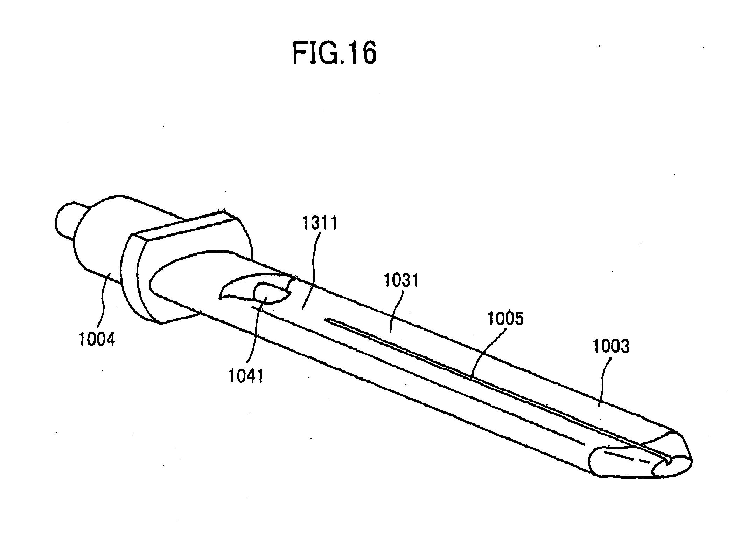

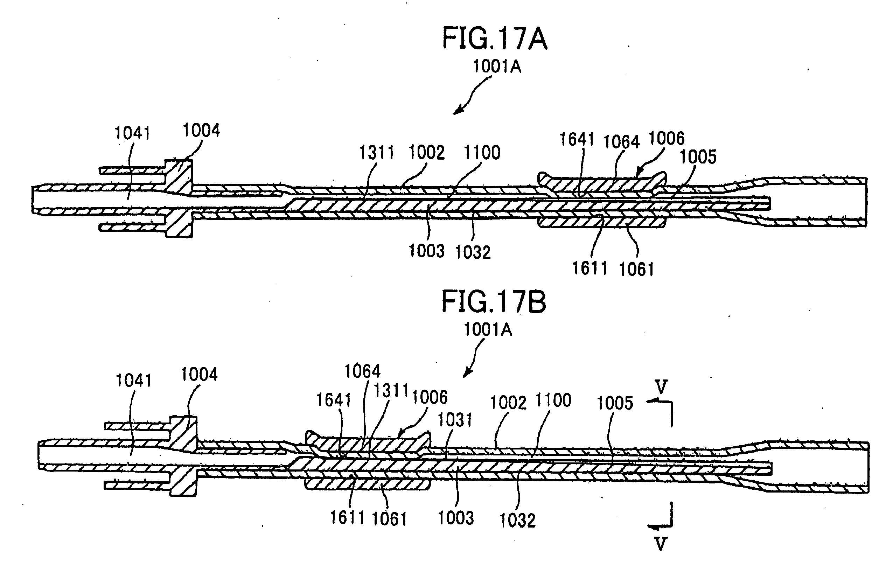

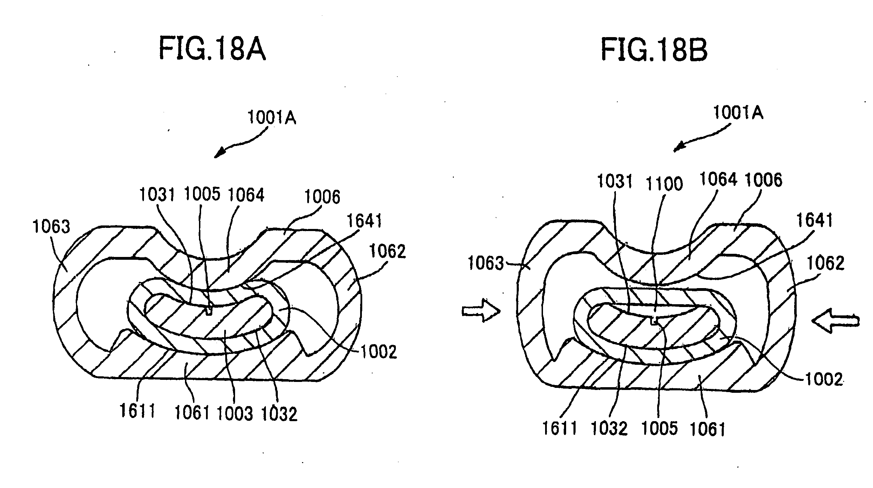

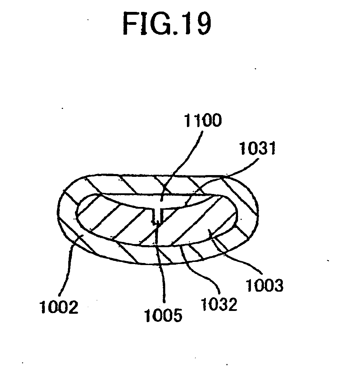

Fig.15 is a perspective view of a flow rate regulator, Fig.16 is a perspective view of a flow passage bottom member integrally equipped with a connection member, Fig. 17 is a longitudinal sectional view showing the operation of the flow rate regulator, Fig. 18 is a cross-sectional view showing the operation of an operating member, and Fig. 19 is a sectional view taken along the line V-V of Fig. 17B. Note that for the convenience of explanation, the axial direction and a direction perpendicular to that direction are defined as described above mentioned. Also, a connection member 1004 side of the flow passage bottom member is called "upstream side", the other side with respect to the upstream side is called "downstream side", the side pressurized by an operating member 1006 or a flow passage bottom member 1003 or the side to which the member is pressurized against is called "pressurized side", and the side opposite to a pressurizing portion is called "the other end side".

As shown in Fig. 15, a flow rate regulator 1001A includes a cylindrical body 1002 forming a flow passage. This cylindrical body 1002 is made of a soft material and is stretchable. Although not shown, the downstream side of the cylindrical body 1002 is connected in a liquid-tight manner to a downstream line such as a liquid infusion or blood transfusion set similarly to the downstream end of the cylindrical body in the flow rate controllers described above.

As shown in Fig. 18, the flow passage bottom member 1003 extending in the axial direction and made of a hard material is arranged inside the cylindrical body 1002. The cylindrical body 1002 is widened in the width direction of the flow passage bottom member 1003 by the flow passage bottom member 1003. A flow passage forming surface (flow passage surface) 1031 of the flow passage bottom member 1003 is formed into a concave configuration, and an adjustment flow passage 1100 is formed between the flow passage forming surface (flow passage surface) 1031 of the flow passage bottom member 1003 and the inner surface of the cylindrical body 1002. Also, another end surface 1032 opposite to the flow passage forming surface (flow passage surface) of the flow passage bottom member 1003 is formed into a convex configuration and is in close contact with the inner surface of the cylindrical body 1002.

Note that the configuration of the cylindrical body 1002 defining an upper portion of the adjustment flow passage 1100 may be convex other than flat as shown in Fig. 19.

As shown in Figs. 16 and 17, the connection member 1004 which is made of a hard material like the flow passage bottom member 1003 is integrally formed to the flow passage bottom member 1003 in the end portion on its upstream side. A hollow tube-like communication channel 1041 extending toward both the upstream side and the downstream side of the connection member 1004 is formed in the connection member 1004. The downstream side of the communication channel communicates with the upstream side of the adjustment flow passage 1100. Although not shown, the upstream side of the communication channel 1041 is connected in a liquid-tight manner to the upstream line of the liquid administering set similarly to the end portion on the upstream side of the connection member in the flow rate controllers described above.

Here, the downstream side of the adjustment flow passage 1100 which is also the downstream side of the cylindrical body 1002 communicates with the downstream line of the liquid administering set, and the upstream side of the adjustment flow passage 1100 communicates with the upstream line of the liquid administering set through the communication channel 1041.

As shown in Fig. 17, a control groove 1005 extending in the axial direction is formed in the flow passage forming surface (flow passage surface) 1031 of the flow passage bottom member 1003. The cross-sectional area of the control groove 1005 is gradually increased from zero toward the downstream side in the axial direction.

The depth of the control groove 1005 is gradually increased in order to gradually expand the cross-sectional area of the control groove 1005, the width of the control groove 1005 or the width and depth of the control groove may be changed. Or conversely, the cross-sectional area of the control groove 1005 may be gradually increased toward the upstream side. The connection member 1004 may be provided on the downstream side or may be provided on both the upstream side and downstream side. Note that as to the control groove 1005, the range of cross-sectional area, the section configuration and the number of control grooves may be set similarly to what is described above.

As shown in Fig. 18, the operating member 1006 made of an elastic material is provided to the outer peripheral portion of the cylindrical body 1002 and can move in the axial direction of the cylindrical body 1002. The operating member 1006 includes a pressurizing portion 1064, and on the other end side of the pressurizing portion 1064, a cylindrical support portion (soft member support portion) 1061 is integrally formed, which supports the cylindrical body 1002 from both sides in cooperation with the other end surface 1032 of the flow passage bottom member 1003. A support surface 1611 of the cylindrical support portion 1061 is formed into a concave configuration in accordance with the configuration of the other end surface 1032 of the flow passage bottom member 1003. Note that the cylindrical support portion 1061 may be provided on the connection member 1004 or the flow passage bottom member 1003 which are independent from the operating member 1006 instead of being formed integrally with the operating member 1006.

The operating member 1006 has opening/closing selection means for selecting either the pressurized state (especially pressure-closed state) of the cylindrical body 1002, or the canceled or eased state. The structure of the opening/closing selection means will be described hereinbelow. As shown in Fig.18, a pair of wall portions 1062 and 1063 which are opposed to each other with the cylindrical body 1002 therebetween (via the cylindrical body 1002) are formed integrally with the cylindrical support portion 1061 of the operating member 1006. The pair of wall portions 1062 and 1063 is respectively formed into a convex arch projecting outward. The pressurizing portion (pressure-closing portion) 1064 is formed in the operating member 1006, for pressure-closing (pressurizing) the cylindrical body 1002 to bring the inner side of the cylindrical body 1002 into close contact with the flow passage forming surface (flow passage surface) 1031 of the flow passage bottom member 1003, and a pressure-closing surface (pressurizing surface) 1641 of the pressurizing portion 1064 is formed into a convex configuration in accordance with the flow passage forming surface (flow passage surface) 1031 of the flow passage bottom member 1003.

The inner side of the cylindrical body 1002 is brought into close contact with the flow passage forming surface (flow passage surface) 1031 of the flow passage bottom member 1003 by pressurizing the cylindrical body 1002 with the pressurizing portion 1064, so that a part of the adjustment flow passage 1100 can be restricted only to the inside of the control groove 1005, that is, to a site covered with the cylindrical body 1002 of the control groove 1005. Accordingly, the flow rate of a fluid passing through the flow rate regulator 1001A is regulated more accurately.

Note that as shown in Fig.17, a shut-off area 1311 devoid of the control groove 1005 is formed on the flow passage forming surface (flow passage surface) 1031 on the upstream side of the control groove 1005 in the flow passage bottom member 1003. When the inner side of the cylindrical body 1002 is brought into close contact with the shut-off area 1311 by pressurizing the vicinity of the upstream end of the cylindrical body 1002 with the pressurizing portion 1064, the adjustment flow passage 1100 is shut off, thereby making it possible to set the flow rate of the infusion liquid to zero.

In addition, the above mentioned pair of wall portions 1062 and 1063 is respectively formed into a convex arch projecting outward as described above mentioned, therefore, as shown in Figs.18A and 18B, when the pair of wall portions 1062 and 1063 are pressurized in a direction for approaching each other, the pair of wall portions 1062 and 1063 elastically deform as such that the pressurizing portion 1064 moves in a direction in which its pressure-closing is canceled or eased (direction in which the pressurizing portion 1064 is spaced apart from the control groove 1005). At the same time, when the pressurizing of the pair of wall portions 1062 and 1063 is canceled, the pressurizing of the pair of wall portions 1062 and 1063 are elastically returned to their original states.

Note that before the pair of wall portions 1062 and 1063 is pressurized in the direction for approaching each other, the width of the operating member 1006 is desirably set to 1.7 to 3.2 times of its vertical length, but the size of the operating member 1006 may be suitably set to enable the above mentioned elastic deformation and elastic return.

The sectional area of the control groove 1005 on its downstream side is set to be large, so that a flushing state can be obtained and maintained. On this downstream side, the cylindrical body 1002 and the flow passage bottom member 1003 are structured as not to come in close contact with each other, the pressure-closing of the pressurizing portion 1064 is canceled and enables the canceled state to be maintained.

Next, regarding the material of each constituent member of the flow rate regulator 1001A, the material of the cylindrical body 1002 is preferably an elastic member that elastically deforms within a certain range, and those enumerated as examples of the material of the cylindrical body in the flow rate regulators described above.

Also, the material of the flow passage bottom member 1003 is preferably a material that has suitable hardness and can form the adjustment flow passage 1100 stably, and those enumerated as examples of the material of the flow passage control member in the flow rate controllers described above.

The material of the operating member 1006 is preferably a material which has suitable hardness enough to deform the cylindrical body 1002, and elastically deforms and elastically returns under the use conditions of the flow rate regulator as described above mentioned, and an example of the material includes a high polymer material such as polypropylene, polyethylene, polyurethane, or fluororesin.

Friction caused by the movement of the operating member 1006 can be reduced and operability can be improved by subjecting the operating member 1006 to anti-rough surface processing, the application of oil such as silicone, the lamination of a film such as PTFE, and other friction reduction treatment.

Next, description will be made of the function of the flow rate regulator.
[1] When the above mentioned liquid infusion bag is installed at a position higher than the arm of a patient, the infusion liquid (fluid) contained in the liquid infusion bag flows or can flow into an injection needle inserted into the vein of the patient through the above mentioned upstream tube, the adjustment flow passage 1100 of the flow rate regulator 1001A, and the above mentioned downstream tube.
[2] In the case of adjusting the flow rate of the infusion liquid in the above mentioned state [1], as shown in Fig. 17A, the operating member 1006 is moved in the axial direction of the flow passage bottom member 1003 and a predetermined position of the cylindrical body 1002 is pressure-closed by the pressurizing portion 1064 of the operating member 1006 such that the inner side at the predetermined position of the cylindrical body 1002 is brought into close contact with the flow passage forming surface (flow passage surface) 1031 (see Fig. 17B) of the flow passage bottom member 1003. Accordingly, the cross-sectional area (capacity) of a position covered with the cylindrical body 1002 of the control groove 1005, namely, the site where the adjustment flow passage 1100 is limited to the control groove 1005 is changed to vary the duct resistance of the adjustment flow passage 1100, thereby making it possible to adjust the flow rate of the infusion liquid to a predetermined level.
   Here, it is possible to make the change amount of the sectional area of the control groove 1005 non-linear and the moving amount of the operating member 1006 at the time of adjusting the flow rate. The accuracy of adjusting the flow rate to a low level can be made higher than the accuracy of adjusting the flow rate to a high level, or the accuracy of adjusting the flow rate to a high level can be made higher than the accuracy of adjusting the flow rate to a low level.
[3] In the case of setting the flow rate of the infusion liquid to zero in the above mentioned state [1], as shown in Fig. 17B, the operating member 1006 is moved toward the upstream side and the vicinity of the upstream end of the cylindrical body 1002 is pressure-closed by the pressurizing portion 1064 such that the inner side of the cylindrical body 1002 in the vicinity of the upstream end is brought into close contact with the shut-off area 1311 of the flow passage forming surface (flow passage surface) 1031 of the flow passage bottom member 1003. Accordingly, the adjustment flow passage 1100 can be shut off to set the flow rate of the infusion liquid to zero.
[4] In the case of temporarily flowing a large amount of the infusion liquid quickly (flushing), as shown in Figs. 18(A) and 18(B), the operator holds the pair of wall portions 1062 and 1063 of the operating member 1006 with fingers and pressurizes them in a direction of approaching each other, to thereby elastically deform the pair of wall portions 1062 and 1063 such that the pressurizing portion 1064 moves in a direction for canceling or relaxing its pressure-closing (direction for spacing apart from the control groove 1005). This function, along with the stretchable function (elastic function) of the cylindrical body 1002, cause the predetermined position of the cylindrical body 1002 to return to a state before it is pressure-closed, and the limitation of part of the adjustment flow passage 1100 is canceled to expand the adjustment flow passage 1100, thereby making it possible to flush the infusion liquid.
[5] In the case of adjusting the flow rate to a predetermined level again after the operation [4] is over (returning the flow rate to a level set in [2] or [3]), cancel the pressurizing of the pair of wall portions 1062 and 1063 of the operating member 1006, to return the pair of wall portions 1062 and 1063 to their original states. Accordingly, the pressurizing portion 1064 of the operating member 1006 moves in a direction for approaching the control groove 1005 and the predetermined position of the cylindrical body 1002 is pressure-closed by the pressurizing portion 1064 again, thereby making it possible to adjust the flow rate of the infusion liquid to the above mentioned predetermined level again.

As described above, in the flow rate regulator 1001A the flow rate of an infusion liquid is adjusted to a predetermined level by changing the cross-sectional area (capacity) of a site covered with the cylindrical body 1002 of the control groove 1005, and thus, the more accurate regulation of the flow rate is made possible and variation in the flow rate of the infusion liquid with passage of time can be restrained after the control of the flow rate of the infusion liquid, thereby enabling the highly accurate control of the flow rate of the infusion liquid. Particularly, as the pressurizing surface 1641 of the operating member 1006 is formed into a convex configuration in accordance with the flow passage forming surface (flow passage surface) 1031 of the flow passage bottom member 1003 as shown in Fig.18, the inner side of the cylindrical body 1002, at a predetermined position, can be brought into firm and close contact with the flow passage forming surface (flow passage surface) 1031 of the flow passage bottom member 1003, thereby further improving the above mentioned effect.

Also, as the operator can cancel the limitation of part of the adjustment flow passage 1100 instantaneously to flush the infusion liquid after it is adjusted, simply by pressurizing the pair of the wall portions 1062 and 1063 of the operating member 1006 in a direction for approaching each other with fingers, the operation of flushing the infusion liquid can be carried out easily and surely.

Further, the flow rate of the infusion liquid can be returned to the original level simply by canceling the pressurizing of the pair of wall portions 1062 and 1063 of the operating member 1006 to pressure-close the predetermined position of the cylindrical body 1002 with the pressurizing portion 1064 of the operating member 1006 again, thereby making it possible to carry out the returning operation easily and surely after the flushing of the infusion liquid. Particularly, as the pair of wall portions 1062 and 1063 of the operating member 1006 is shaped like a convex arch projecting outward, the elastic deformation function and elastic returning function of the operating member 1006 can be promoted, thereby further improving the above mentioned effect.

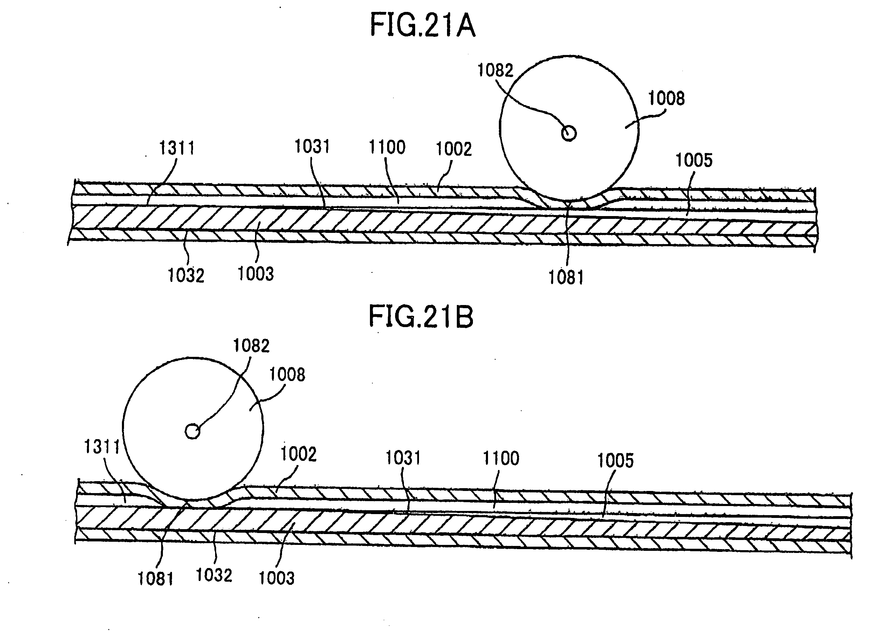

Fig.20 is a perspective view of a further flow rate regulator, Fig.21 is a longitudinal sectional view showing an operation of the latter flow rate regulator, and Fig.22 is a cross-sectional view showing the operation of an operating member.

A flow rate regulator 1001B is composed of, the cylindrical body 1002, flow passage bottom member 1003, and control groove 1005 which are the same constituent elements as in the flow rate regulator 1001A. In place of the operating member 1006 having the pressurizing portion 1064 in the flow rate regulator 1001A, an operating member is composed of an operation support member 1007 and a pressure-closing roller 1008. Note that the flow rate regulator 1001B may include a member having the same function as that of the above mentioned connection member 1004.

The characteristic portions of the flow rate regulator 1001B will be mainly described hereinbelow. Note that detailed description of the structures of the cylindrical body 1002, flow passage bottom member 1003, and control groove 1005 will be omitted.

The operation support member 1007 is made of an elastic material and cannot move in the axial direction of the flow passage bottom member 1003 by being mounted to the cylindrical body 1002 so as to be fixed thereto. As shown in Fig. 20, one side of the substantially annular operation support member 1007 is open and is composed of the pressure-closing roller 1008 for pressurizing the cylindrical body 1002 in such a manner that it can move in the axial direction of the flow passage bottom member 1003. On the other end of closed side surface, a cylindrical body support portion (soft member support portion) 1071 for supporting the cylindrical body 1002 from both sides in cooperation with the other end surface 1032 of the flow passage bottom member 1003 is formed integrally so as to project inward. A support surface 1711 of the cylindrical body support portion 1071 is formed into a concave configuration in accordance with the other end surface 1032 of the flow passage bottom member 1003.

A pair of wall portions 1072 and 1073, which is opposed to each other with the cylindrical body 1002 therebetween, is formed integrally with the cylindrical body support portion 1071 of the operation support member 1007. The pair of wall portions 1072 and 1073 is respectively formed into a convex arch projecting outward. Guide grooves 1721 and 1731 extending in the axial direction of the flow passage bottom member 1003 are formed on the inner surfaces of the pair of wall portions 1072 and 1073, respectively.

A shaft 1082, of the pressure-closing roller 1008 for pressure-closing the cylindrical body 1002 to bring the inner side of the cylindrical body 1002 into close contact with the flow passage forming surface (flow passage surface) 1031 of the flow passage bottom member 1003, is inserted into the pair of guide grooves 1721 and 1731. Accordingly, the pressure-closing roller 1008 is supported so as to be rotatable and movable in the axial direction of the flow passage bottom member 1003. A pressure-closing surface (pressurizing surface) 1081, which is the outer peripheral surface of the pressure-closing roller 1008, is formed into a convex configuration in accordance with the flow passage forming surface (flow passage surface) 1031 of the flow passage bottom member 1003. The inner surface of the cylindrical body 1002 is brought into close contact with the flow passage forming surface (flow passage surface) 1031 of the flow passage bottom member 1003 by pressurizing the cylindrical body 1002 with the pressure-closing roller 1008, so that a part of the above mentioned adjustment flow passage 1100 can be limited to a site covered with the cylindrical body 1002 of the control groove 1005.

As shown in Fig. 20, a connection tube 1074 which communicates with the adjustment flow passage 1100 is provided on the upstream side of the operation support member 1007 and connected in a liquid-tight manner to the upstream line of the liquid administering set, although not shown.

Further, the facts, that the side where the pressure-closing roller 1008 of the operation support member 1007 operates is open, and a pair of wall portions 1072 and 1073 is respectively formed into a convex arch projecting outward as described above, work effectively, when the pair of wall portions 1072 and 1073 are pressurized in a direction for approaching each other, as shown in Fig. 22A and 22B, the pair of wall portions 1072 and 1073 elastically deform such that the pressure-closing roller 1008 moves in a direction in which its pressure-closing is canceled or eased (direction shown by an arrow of Fig. 22B). At the same time, when the pressurizing of the pair of wall portions 1072 and 1073 is canceled, the pair of wall portions 1072 and 1073 is elastically returned to their original states.

The material of the operation support member 1007 is preferably a material which elastically deforms and elastically returns as described above. An example of the material includes a high polymer material such as polypropylene, polyethylene, polyurethane, and fluororesin.

The material of the pressure-closing roller 1008 is preferably a material having suitable hardness enough to deform the cylindrical body 1002. An example of the material includes a high polymer material such as polycarbonate, polypropylene, and high-density polyethylene.

Next, description will be made of the function of the flow rate regulator 1001B.
[1] When the above mentioned liquid infusion bag is installed at a position higher than the arm of a patient, the infusion liquid (fluid) contained in the liquid infusion bag flows or can flow into an injection needle inserted into the vein of the patient through the upstream tube, the adjustment flow passage 1100 of the flow rate regulator 1001B, and the downstream tube.
[2] In the case of adjusting the flow rate of the infusion liquid in the above mentioned state [1], as shown in Fig. 21(A), an operator rotate the pressure-closing roller 1008 to moves in the axial direction of the flow passage bottom member 1003 while turning the pressure-closing roller 1008 with the finger or the like to pressure-close (press) a predetermined position of the cylindrical body 1002 with the pressure-closing roller 1008, thereby bringing the inner side at the predetermined position of the cylindrical body 1002 into close contact with the flow passage forming surface (flow passage surface) 1031 of the flow passage bottom member 1003. Accordingly, the cross-sectional area (capacity) of a site covered with the cylindrical body 1002 of the control groove 1005, namely, the site where the adjustment flow passage 1100 is limited to the control groove 1005 is changed to vary the duct resistance of the adjustment flow passage 1100, thereby making it possible to adjust the flow rate of the infusion liquid to a predetermined level.
[3] In the case of setting the flow rate of the infusion liquid to zero in the above mentioned state [1], as shown in Fig.21 (B), the operator moves the pressure-closing roller 1008 toward the upstream side while turning the pressure-closing roller 1008 with the finger or the like, to thereby pressure-close the vicinity of the end portion on the upstream side of the cylindrical body 1002 with the pressure-closing roller 1008 so as to bring the inner side of the vicinity of the upstream end of the cylindrical body 1002 into close contact with the shut-off area 1311 of the flow passage forming surface (flow passage surface) 1031 of the flow passage bottom member 1003. Accordingly, the adjustment flow passage 1100 is shut off to set the flow rate of the infusion liquid to zero. When the pressure-closing roller 1008 is moved to the end portion on the downstream side, the flow rate of the infusion liquid can be maximized.
[4] In the case of temporarily flowing a large amount of the liquid quickly (flushing), as shown in Figs. 22(A) and 22(B), the operator holds the pair of wall portions 1072 and 1073 of the operation support member 1007 with the fingers and pressurizes them into a direction for approaching each other, to thereby elastically deform the pair of wall portions 1072 and 1073 such that the pressure-closing roller 1008 moves in a direction in which its pressure-closing is canceled or eased (direction for spacing apart from the control groove 1005). This function and the stretchable function (elastic function) of the cylindrical body 1002 cause the predetermined position of the cylindrical body 1002 to return to a state before it is pressure-closed, and the limitation of part of the adjustment flow passage 1100 is canceled to expand the adjustment flow passage 1100, thereby making it possible to flush the infusion liquid.
[5] In the case of adjusting the flow rate to a predetermined level again after the operation [4] is over (returning the flow rate to a level set in [2] or [3]), the pressurizing of the pair of wall portions 1072 and 1073 of the operation support member 1007 is canceled to return the pair of wall portions 1072 and 1073 to their original states. Accordingly, the pressure-closing roller 8 moves in a direction for approaching the control groove 1005 and the predetermined position of the cylindrical body 1002 is pressure-closed by the pressure-closing roller 1008 again, thereby making it possible to adjust the flow rate of the infusion liquid to the above mentioned predetermined level again.

As described above, in the flow rate regulator 1001B, similarly to the effect of the flow rate regulator 1001A, the more accurate regulation of the flow rate is possible and variation with passage of time in the flow rate of the infusion liquid from a predetermined adjusted level can be restrained, thereby enabling the highly accurate control of the flow rate of the infusion liquid.

Also, when the operator simply pressurizes the pair of wall portions 1072 and 1073 of the operation support member 1007 in a direction for approaching each other with the fingers after the flow rate of the liquid has been adjusted, the limitation of part of the adjustment flow passage 1100 is canceled instantaneously, thereby making it possible to flush the liquid, thus the operation of flushing the infusion liquid can be carried out easily and surely.

Further, the flow rate of the infusion liquid can be returned to the original level simply by canceling the pressurizing of the pair of wall portions 1072 and 1073 of the operation support member 1007 to pressure-close the predetermined position of the cylindrical body 1002 with the pressure-closing roller 1008 again, thereby making it possible to carry out the returning operation easily and surely after the flushing of the liquid. Particularly, the side where the pressure-closing roller 1008 is operated of the operation support member 1007 is open, and the pair of wall portions 1072 and 1073 of the operation support member 1007 are shaped like a convex arch projecting outward, the elastic deformation function and elastic returning function of the operation support member 1007 can be promoted, thereby further improving the above mentioned effect.

Further, when the cylindrical body support portion 1071 is formed integrally with the operation support member 1007, the number of parts of the flow rate regulator 1001B can be reduced, so that the structure of the flow rate regulator 1001B can be simplified and the cost of the flow rate regulator 1001B can be cut.

Also, the number of parts can be reduced and the cost can be cut by molding the support member 1007 and the flow passage bottom member 1003 integrally.

Also, the operator can move the pressure-closing roller 1008 in the axial direction of the flow passage bottom member 1003 by turning the pressure-closing roller with the finger or the like, so that the control of the flow rate of the infusion liquid can be carried out more easily and the fine control of the flow rate can be effected advantageously.

Fig. 23 is a perspective view of a further flow rate regulator, and Fig. 24 is a perspective view of a pressure-closing slider.

A flow rate regulator 1001C includes the cylindrical body 1002, the flow passage bottom member 1003, the control groove 1005, and the operation support member 1007 which are the same constituent elements as in the flow rate regulator 1001B, but in place of the above mentioned pressure-closing roller 1008, a pressure-closing slider 1009 is provided. That is, the operating member is composed of the operation support member 1007 and the pressure-closing slider 1009.

The characteristic portions of the flow rate regulator 1001C will be mainly described hereinbelow. Note that detailed description of the structures of the cylindrical body 1002, flow passage bottom member 1003, control groove 1005, and operation support member 1007 will be omitted.

Similarly to the operating member 1072 of the flow rate regulator 1001B as shown in Fig. 22, the pair of guide grooves 1721 and 1731 are formed on the inner sides of opening side surfaces of the operation support member 1007. A pair of ribs 1093 and 1093 of the pressure-closing slider 1009 shown in Figs.23 and 24 is inserted into the guide grooves 1721 and 1731 of the operating member 1071, respectively, and the pressure-closing slider 1009 is supported so as to be movable in the axial direction of the flow passage bottom member 1003. The pressure-closing slider 1009 pressure-closes the cylindrical body 1002, and brings the inner side of the cylindrical body 1002 into close contact with the flow passage forming surface (flow passage surface) 1031 of the flow passage bottom member 1003.

As shown in Fig.24, a pressure-closing surface (pressurizing surface) 1091 of the pressure-closing slider 1009 is formed into a convex configuration (curved convex surface) in accordance with a concave configuration of the flow passage forming surface (flow passage surface) 1031 of the flow passage bottom member 1003. Here, the inner side of the cylindrical body 1002 is brought into close contact with the flow passage forming surface (flow passage surface) 1031 of the flow passage bottom member 1003 by pressurizing the cylindrical body 1002 with the pressure-closing slider 1009, so that a part of the adjustment flow passage 1100 can be restricted only to a site covered with the cylindrical body 1002 of the control groove 1005.

As shown in Fig. 23, a scale 1722 is formed on the outer side of the wall portion 1072 of the opening side surface in the operation support member 1007 along the axial direction of the flow passage bottom member 1003, while an indicator line 1092 for aligning with a predetermined position in the scale 1722 is formed on the pressure-closing slider 1009. The displacement amount of the pressure-closing surface (pressurizing portion) 1091 is indicated by the scale 1722, and therefore the pressure-closing surface 1091 of the pressure-closing slider 1009 can be moved to a desired position in the axial direction of the flow passage bottom member 1003, thereby enabling the more accurate adjustment of the flow rate. Note that instead of forming the scale 1722 on one wall portion 1072, a similar scale may be formed on the cylindrical body 1002 or the flow passage bottom member 1003.

As described above, the fact that one side surface of the operation support member 1007 is opened together with the pair of wall portions 1072 and 1073 is respectively formed into a convex arch projecting outward, help the pair of wall portions 1072 and 1073 elastically deform in such that the pressure-closing slider 1009 moves in a direction in which to cancel its pressure-closing. When the pressurizing of the pair of wall portions 1072 and 1073 is canceled, the pair of wall portions 1072 and 1073 is elastically returned to its original state.

The material of the pressure-closing slider 1009 is preferably a material, which has suitable hardness enough to deform the cylindrical body 1002, such as a high polymer material like polycarbonate, polypropylene, or high-density polyethylene.

The function of the flow rate regulator 1001C is almost the same as that of the flow rate regulator 1001B and therefore its description will be omitted.

According to the flow rate regulator 1001C, similarly to the effect of the flow rate regulator 1001B, the more accurate regulation of the flow rate is possible and variation with passage of time in the flow rate of the infusion liquid from a predetermined adjusted level can be restrained, thereby enabling the highly accurate control of the flow rate of the infusion liquid. Also, both the operation of flushing the infusion liquid and the returning operation after the flushing can be carried out quickly, easily and surely. Further, the structure of the flow rate regulator 1001C can be simplified and the cost of the flow rate regulator 1001C can be cut.

Also, as the cylindrical body 1002 is pressure-closed by a surface pressure of the pressure-closing surface 1091 having a certain length in the axial direction, the site of the cylindrical body 1002, which is pressure-closed by the pressure-closing slider 1009, is hardly changed in size, by variation of a pressure-closing pressure generated when the pressure-closing slider 1009 moves in the axial direction of the flow passage bottom member 1003, thereby enabling the stable adjustment of the flow rate of the infusion liquid.

In the described flow rate regulator, the soft member composing the cylindrical body 1002 is not limited to the cylindrical configuration as described above, if the soft member has the same function, and may have, for example, a plate-like configuration (sheet-like configuration). And although a portion pressurized by the pressurizing portion needs to be a soft member that elastically deforms, other sites may be formed of hard materials. Therefore, as shown in Fig. 25, the flow passage (adjustment flow passage 1100) may be defined by a cylindrical member 1021 composed of a membrane-like portion 1023 that has a plate-like configuration and is formed of a soft member and a flow passage bottom member 1030.

Fig. 25 is a sectional view showing another structural example of the soft member composing the cylindrical body. The cylindrical member 1021 shown in the drawing is composed of the membrane-like portion 1023, which has a plate-like configuration (sheet-like configuration) formed of a soft member, and the flow passage bottom member 1030 formed of a hard member. The membrane-like portion 1023 is joined, as mentioned above, onto the flow passage bottom member 1030, having the control groove 1005, and passage forming surface (flow passage surface) 1031, and is press-contacted and fixed by a fixing member 1033.

The fixing member 1033 includes a pair of claw portions 1034 and 1034, a pair of press-contact ribs 1035 and 1035, and an opening portion 1036. Both of the claw portions 1034 are engaged to a lower surface edge portion of the flow passage bottom member 1030, and the press-contact ribs 1035 are press-contacted so as to pressurize the membrane-like portion 1023 against the upper surface of the flow passage bottom member 1030. Accordingly, the membrane-like portion 1023 is fixed in a liquid-tight manner to the flow passage bottom member 1030.

By inserting, from the opening portion 1036 of the fixing member 1033, the pressurizing portion 1064, the pressure-closing roller 1008, or the pressure-closing slider 1009 included in the flow rate controller and the flow rate regulators according to the first to eighth embodiments mentioned above, and by pressurizing the membrane-like portion 1023 downward in Fig. 25 (in the direction for approaching the control groove 1005), a partial flow passage of the adjustment flow passage 1100 can be limited to the inside of the control groove 1005, that is, to a site covered with the membrane-like portion 1023 of the control groove 1005, thus the flow rate is regulated more accurately.

Note that the membrane-like portion 1023 may be directly fixed to the flow passage bottom member 1030 by a method such as adhesion or fusion without using the fixing member 1033.

Note that the flow rate regulator 1001A and the flow rate regulator 1001B can each be provided with a scale similarly to the flow rate regulator 1001C.

Further, the respective flow rate regulators may have structures in which a positioning concave portion and a positioning convex portion which can be engaged with each other are suitably provided such that the positions of the operating member 1007, the operating member 1006, the pressure-closing roller 1008, and the pressure-closing slider 1009 can be determined particularly in the axial direction.

Further, the flow rate regulator 1001A, 1001B, 1001C may be incorporated into the liquid administering set such as the liquid infusion set in reverse to the direction as described above.

Further, in the case of incorporating the flow rate regulator into the liquid administering set before use, the flow rate regulator may be connected not only to the liquid infusion set but also to another set such as a set for administering a nutritional liquid.

Next, description will be made of a flow rate switcher according to the present invention. Similarly to the flow rate control device and the flow rate regulator (controller) described above, the flow rate switcher of this embodiment is incorporated into a liquid administering set such as a liquid infusion set, a blood transfusion set, and a nutritional transfusion set before use.

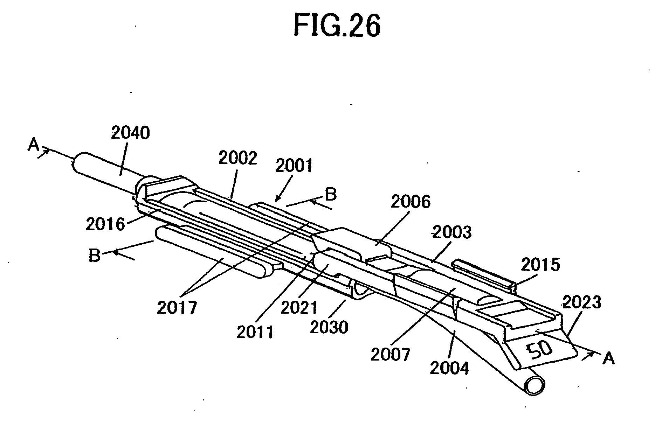

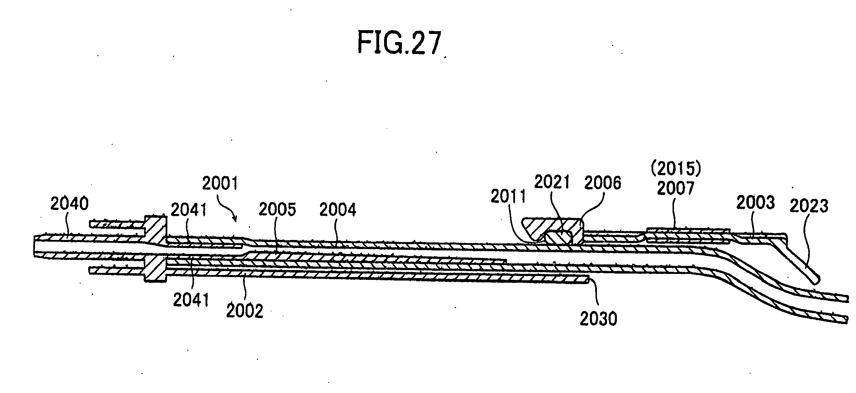

Fig. 26 is a perspective view of the flow rate switcher according to the present invention. Figs. 27, 28, and 29 are sectional views of the flow rate switcher taken along the line A-A of Fig. 26, and show an operating member 2003 in differently turned states;

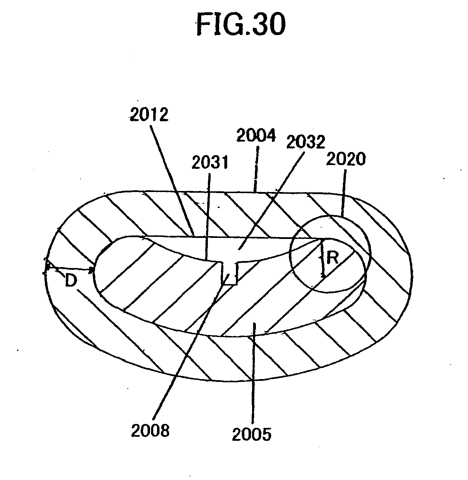

Fig. 30 is a sectional view of the flow rate switcher according to the present invention taken along the line B-B of Fig. 26, and shows only a cylindrical body 2004 and a flow passage bottom member 2005 placed in an interior of the cylindrical body in the structure of the flow rate switcher. Note that for convenience of explanation, the axial direction and a direction perpendicular to that direction are defined as described above. Also, as shown in Figs.26 to 29, the side having an upstream connection portion 2040 of a flow rate switcher 2001 which is connected to the upstream line of the liquid administering set or the like provided to the upstream side of the flow rate switcher of a support body 2002 is called "upstream side", and the other side with respect to the upstream side is called "downstream side".

The flow rate switcher 2001 according to the present invention is composed at least of the support body 2002, the operating member 2003, the cylindrical body 2004, and the flow passage bottom member 2005.

As shown in Fig.27, the support body 2002, having a plate-like configuration or semi-circular configuration and extending in the axial direction, includes a cavity portion for receiving the cylindrical body 2004 inside. The end portion on the upstream side of the support body 2002 is provided with the upstream connection portion 2040 having a hollow tube structure. The cylindrical body 2004 received in the support body 2002 is connected in a liquid-tight manner to a cylindrical body connection port 2041 provided on the downstream side of the upstream connection portion 2040. The cylindrical body connection port 2041 is molded integrally with the flow passage bottom member 2005. However, the upstream connection portion 2040, provided on the upstream side of the support body 2002, is not always necessary. The support body 2002 may have an opening portion on the upstream side having the same configuration as that of an end portion 2030 of the support body 2002 on the downstream side, that is, having the same section configuration as the configuration of the cavity portion inside the support body 2002, and may be provided with another member for connecting to the upstream line.

The cylindrical body 2004, comprising of an inlet and an outlet port for a fluid, is situated between the support body 2002 and the operating member 2003, respectively extending in the axial direction. The cylindrical body 2004 is received in the cavity portion inside the support body 2002, and part of the outer periphery of the cylindrical body 2004 is supported by the inner side surface of the support body. The cylindrical body 2004 forming the flow passage of the fluid is formed of a soft member and is stretchable. Although not shown, the upstream side of the cylindrical body 2004 is connected in a liquid-tight manner to the upstream line of a medical instrument such as a liquid infusion bag, a blood transfusion bag, or a nutrition bag through the upstream connection portion 2040 of the support body 2002 similarly to that of the cylindrical body in the flow rate controllers and the flow rate regulators.

Although not shown, the downstream side of the cylindrical body 2004 is connected in a liquid-tight manner to the downstream line of the medical instrument such as a liquid infusion bag, a blood transfusion bag, or a nutrition bag similarly to that of the cylindrical body in the flow rate controllers and the flow rate regulators.

Further, the cylindrical body 2004 itself may be a liquid transfusion tube, a blood transfusion tube, a nutritional transfusion tube, or the like. Thus, the manufacturing cost thereof can be further suppressed.

As shown in Fig.30, the flow passage bottom member 2005 made of a hard material is arranged in the interior of the cylindrical body 2004, and part of the interior surface of the cylindrical body 2004 and part of the outer surface of the flow passage bottom member 2005 are closely fixed to each other so as not to pass the fluid. Between the cylindrical body 2004 and a flow passage forming surface 2031 of the flow passage bottom member 2005, an adjustment flow passage 2032 is formed.

As shown in Fig. 27, the flow passage bottom member 2005 exists in the part of the interior, of a portion of the cylindrical body 2004 supported by the support body 2002. That is, in the portion supported by the support body 2002 of the cylindrical body 2004, there exist a portion where the flow passage bottom member 2005 is disposed in its interior and a portion where the flow passage bottom member 2005 is not disposed in its interior.

As shown in Fig.27, the operating member 2003 extending in the axial direction has a second pressurizing portion 2011 provided at its end portion on the upstream side and a grip portion 2023 provided at its end portion on the downstream side. A first pressurizing portion 2007 is provided between the second pressurizing portion 2011 and the grip portion 2023 of the operating member 2003. In the drawing, the second pressurizing portion 2011 forms the end portion on the upstream side of a rotating portion 2021, formed into an oval configuration, on the upstream side of the operating member 2003.

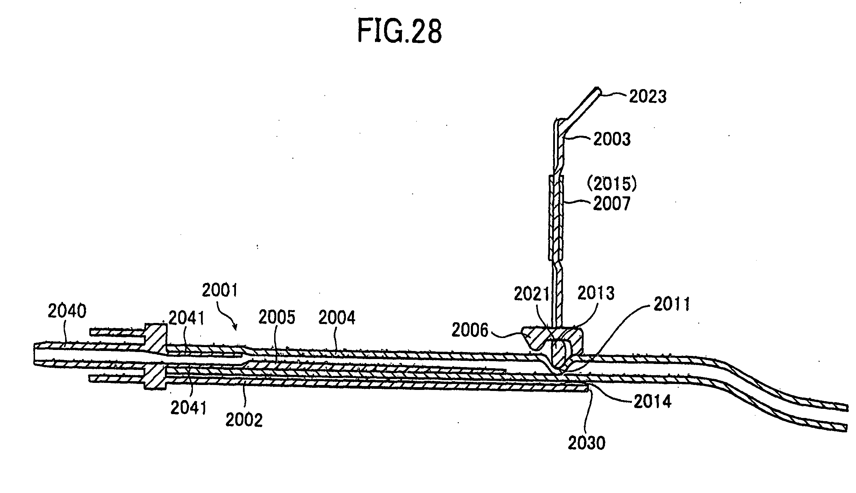

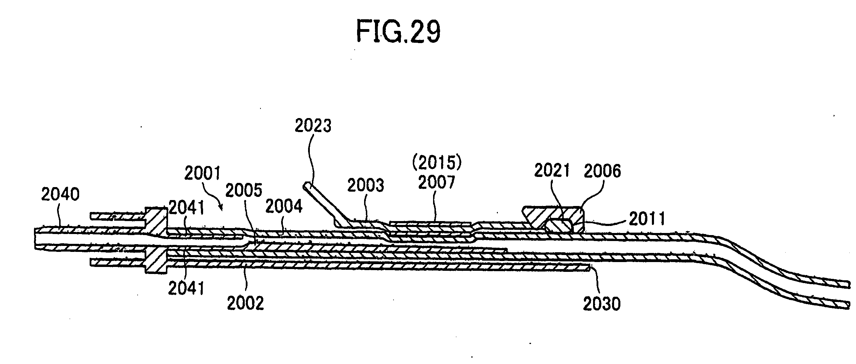

The operating member 2003 is coupled to the support body 2002 by engaging the rotating portion 2021, formed into an oval configuration on the upstream side of the operating member 2003, with a substantially L-shape engagement portion 2006 provided on the downstream side of the support body 2002. Accordingly, the operating member 2003 can be turned perpendicularly to the support body 2002 using the engagement portion 2006 as a fulcrum. That is, the operating member 2003 is turned from a state shown in Fig. 27 to a state shown in Fig. 28, and further to a state shown in Fig. 29. In the state shown in Fig. 27, the second pressurizing portion 2011 is situated on the upstream side, the grip portion 2023 is situated on the downstream side, and the operating member 2003 is arranged substantially parallel to the cylindrical body 2004. In the state shown in Fig.. 28, the operating member 2003 is arranged perpendicularly to the cylindrical body 2004, and the second pressurizing portion 2011 contacts the flow passage side outer peripheral portion of the cylindrical body 2004 to pressurize the flow passage side outer peripheral portion. In the state shown in Fig. 29, the second pressurizing portion 2011 is situated on the downstream side, the grip portion 2023 is situated on the upstream side, the operating member 2003 is arranged substantially parallel to the cylindrical body 2004, and the first pressurizing portion 2007 formed on the operating member 2003 pressurizes the flow passage side outer peripheral portion of the cylindrical body 2004. Thus, the upstream side and the downstream side of the operating member 2003 are reversed. Therefore, by turning the operating member 2003 using the engagement portion 2006 as a fulcrum, only one pressurizing portion, the first pressurizing portion 2007 or the second pressurizing portion 2011 formed on the operating member 2003, pressurizes the flow passage side outer peripheral portion of the cylindrical body 2004.

The first pressurizing portion 2007 of the operating member 2003 pressurizes the flow passage side outer peripheral portion of the portion of the cylindrical body 2004 where the flow passage bottom member 2005 is disposed in its interior in cooperation with the flow passage bottom member 2005. As a result, although described in detail later, the adjustment flow passage 2032 in the interior of the cylindrical body 2004 is limited to a control groove 2008 alone, and the flow rate of the fluid passing through the flow rate switcher 2001 is adjusted to a preset flow rate. On the other hand, the second pressurizing portion 2011 of the operating member 2003 pressurizes the flow passage side outer peripheral portion of the cylindrical body 2004, where the flow passage bottom member 2005 is not disposed in its interior, in cooperation with the support body 2002. As a result, the flow passage is completely closed and the flow rate of the fluid becomes zero.

As shown in Fig. 30, the control groove 2008 extending in the axial direction of the cylindrical body 2004 is formed in the flow passage forming surface 2031 of the flow passage bottom member 2005, and the cross-sectional area of the control groove 2008 is suitably set so as to control the flow rate of the fluid. The control groove 2008 may have a cross-sectional area varied due to the change in width and/or depth of the control groove 2008 along the axial direction of the cylindrical body 2004. Note that the cross-sectional area and the section configuration of the control groove 2008 may be set similarly to the cross-sectional area and the section configuration of the control groove of the other devices described above.

The section configuration of both side end portions 2020 of the flow passage bottom member in the vicinity of the boundary between the cylindrical body 2004 and the flow passage bottom member 2005 has a continuous, gentle roundness. Accordingly, when the cylindrical body 2004 is pressurized by the first pressurizing portion 2007, the flow passage forming surface 2031 of the flow passage bottom member 2005 and the cylindrical body 2004 are brought into close contact with each other excluding the control groove 2008.

That is, the interior of the cylindrical body 2004 is in a state where only the control groove 2008 is open, and the adjustment flow passage 2032 is limited to the control groove 2008 alone. In this case, by suitably setting the cross-sectional area of the control groove 2008 as described above, the flow rate of the fluid passing through the flow rate switcher 2001 of the present invention is determined.

Similarly to the relationship between the pressurizing portion and the flow passage control member or the flow passage bottom member in the flow rate controllers and the flow rate regulators(controllers), the section configuration of the flow passage bottom member 2005, as taken perpendicularly to the axial direction of the cylindrical body 2004, is a concave configuration, and a member for pressurizing the cylindrical body 2004 provided to the first pressurizing portion 2007 has a section configuration of a convex configuration large enough to be inserted into the concave configuration. The relationship between a radius R of the continuous, gentle roundness in the section configurations of the both side end portions of the flow passage bottom member 2005 shown in Fig.30, and a thickness D of the cylindrical body, is preferably represented as R ≥ D/10.

If the relationship between the radius R of the section configurations of the both side end portions of the flow passage bottom member 2005, and the thickness D of the cylindrical body 2004 satisfies R ≥ D/10, the continuous, gentle roundness can be attained. If the relationship between the radius R and the thickness D is R < D/10, it becomes difficult to bring the both side end portions of the cylindrical body 2004 into close contact with the both side end portions of the flow passage bottom member 2005 due to their configurations, when the first pressurizing portion 2007 pressurizes the cylindrical body 2004, thus occurring a gap between the flow passage bottom member 2005 and the cylindrical body 2004.

The cylindrical body 2004 includes a deformable portion (deform portion) 2012 in which the pressurized site deforms at the time of pressurization. When the first pressurizing portion 2007 pressurizes the outer peripheral portion of the cylindrical body 2004, the deformable portion (deform portion) 2012 of the cylindrical body 2004 deforms, and the deformable portion (deform portion) 2012 and the flow passage forming surface 2031 of the flow passage bottom member 2005 are brought into close contact with each other excluding the control groove 2008.

It is preferable that a tensile strength of zero or more be constantly applied to the deformable portion (deform portion) 2012 in the inner side of its section taken perpendicularly to the axial direction of the cylindrical body 2004. If the tensile strength of zero or more is constantly applied, there occurs no sag in the deformable portion (deform portion) 2012, in the non-pressurized state. Accordingly, when pressurized by the first pressurizing portion 2007, the deformable portion 2012 (deform portion) is less likely to involve generation of a gap between the deformable portion and the sites corresponding to the gently rounded curved surface of the flow passage bottom member 2005.

The method of applying the tensile strength is not particularly limited, but for example, the tensile strength can preferably be applied by setting the peripheral length of the section configuration of the flow passage bottom member 2005 to be slightly larger than the circumferential length of the interior of the cylindrical body 2004.

Further, it is preferable that in the non-pressurized state, the section configuration as taken perpendicularly to the axial direction of the cylindrical body 2004 be linear, and that the vicinity of the surface where the interior of the cylindrical body 2004 contacts the flow passage bottom member 2005 in the vicinity of the adjustment flow passage 2032, formed of the deform portion 2012 and the flow passage bottom member 2005, be a close contacted portion, in which the interior of the cylindrical body 2004 and the flow passage bottom member 2005 is not fixed to but in close contact with each other. With this arrangement, when the first pressurizing portion 2007 pressurizes the cylindrical body 2004, a gap is less likely to be generated between the interior of the cylindrical body 2004 and the gently rounded curved surface of the flow passage bottom member 2005.

As shown in Fig.26, the first pressurizing portion 2007 of the operating member 2003 has a certain length in the axial direction and a convex configuration. Thus, when the flow passage side outer peripheral portion of the cylindrical body, where the flow passage bottom member 2005 is disposed in its interior, is pressurized by the first pressurizing portion 2007, the first pressurizing portion 2007 having a convex configuration, and the concave portion of the flow passage bottom member 2005, are brought into close contact with each other so that the adjustment flow passage 2032 inside the cylindrical body 2004 is limited to the control groove 2008 alone. Also, the first pressurizing portion 2007 has a certain length in the axial direction, to thereby function as the resistance with respect to the fluid passing through the control groove 2008. As a result, the flow rate of the fluid passing through the flow rate switcher 2001 is adjusted to a desired flow rate.

However, in the flow rate switcher of the present invention, the first pressurizing portion 2007 is not necessarily be limited to the form shown in Fig.26. For example, in flow rate regulators, the first pressurizing portion 2007 may be a pressurizing portion such as the roller shown in Fig. 22 or the slider shown in Figs. 23 and 24, which can move in the axial direction of the cylindrical body to thereby enable selection of the flow rate of the fluid in different levels. If the pressurizing portion described above is used in combination with the control groove 2008 having a cross-sectional that varies along the axial direction of the flow passage bottom member 2005, while the flow passage side outer peripheral portion of the cylindrical body 2004 is pressurized by the first pressurizing portion 2007, it is further possible to suitably set the flow rate of the fluid.

Further, the flow rate switcher 2001 of the present invention may include the operating member 2003 which is detachable. If the operating member 2003 is detachable, the set flow rate of the flow rate switcher 2001 can be changed suitably, by preparing a plurality of operating members 2003 which differ from each other in the axial length of the first pressurizing portion 2007 and in the position where the first pressurizing portion 2007 is disposed on the operating member 2003. In the case of using the operating members 2003 provided with the first pressurizing portions 2007 which differ in the axial length of the pressurizing surface from each other, the passage resistance with respect to the fluid passing through the control groove 2008 is changed, thereby changing the set flow rate of the flow rate switcher 2001. When the operating members 2003, which differ in the position of the first pressurizing portion 2007 from each other, are used in combination with the control groove 2008 having a cross-sectional that varies along the axial direction of the cylindrical body 2004, the cross-sectional area of the control groove 2008 is changed to thereby alter the set flow rate of the flow rate switcher 2001, when the adjustment flow passage 2032 is limited to the control groove 2008 alone.

Further, the flow rate switcher 2001 of the present invention may include retaining means for retaining a state where the flow passage side outer peripheral portion of the cylindrical body 2004 is pressurized by the first pressurizing portion 2007. In the case of the flow rate switcher 2001 shown in Fig.26, a claw portion 2015 provided to the operating member 2003 is engaged with a protruding portion 2016 provided to the support body 2002, so that the flow passage side outer peripheral portion of the cylindrical body 2004 can be kept in the state where it is pressurized by the first pressurizing portion 2007. To cancel this state, an operating portions 2017 provided to the both side end portions of the operating member 2003 are pressurized simultaneously, thus the engagement between the protruding portion 2016, provided to the support body 2002, and the claw portion 2015, provided to the operating member 2003, becomes eased. So by holding and turning the grip portion 2023 provided to the end portion of the operating member 2003 at this time, the engagement between the claw portion 2015 and the protruding portion 2016 can be canceled with ease.

The second pressurizing portion 2011 of the operating member 2003 pressurizes the flow passage side outer peripheral portion of the cylindrical body 2004 where the flow passage bottom member 2005 is not disposed in its interior, in cooperation with the support body 2002. Thus, the deformable portion (deform portion) 2012 of the cylindrical body 2004 deforms toward the support body 2002 side. And thus, the deformable portion (deform portion) 2012 is brought into close contact with the other inner surface of the cylindrical body 2004, so that the flow passage inside the cylindrical body 2004 is completely closed.

When the grip portion 2023 in Fig.27 is operated to turn the operating member 2003 in a direction perpendicular to the support body 2002, as shown in Fig. 28, the operating member 2003 is arranged perpendicularly to the cylindrical body 2004, thus the second pressurizing portion 2011, formed to the end portion of the rotating portion 2021, is brought into contact with the flow passage side outer peripheral portion of the cylindrical body 2004 where the flow passage bottom member 2005 is not disposed in its interior, and pressurizes the flow passage side outer peripheral portion.

When the flow passage side outer peripheral portion of the cylindrical body 2004, where the flow passage bottom member 2005 is not disposed in its interior, is pressurized by the second pressurizing portion 2011, in order to completely close the flow passage of the interior of the cylindrical body 2004, as shown in Fig. 28, it is preferable that the length in the long axial direction of the rotating portion 2021, which has an oval configuration, be the same as or slightly shorter than a length, which is measured from the top surface of the substantially L-shaped engagement portion 2006 arranged on the downstream side of the support body 2002, in other words, a top surface 2013 that comes in contact with the rotating portion 2021 when the outer peripheral portion of the cylindrical body 2004 is pressurized by the second pressurizing portion, to the inner side surface of the support body 2002, in other words, a bottom surface 2014 to which the cylindrical body 2004 is closely fixed. If the length in the long axial direction of the rotating portion 2021 is thus set, the rotating portion 2021 is sandwiched between the top surface 2013 and the bottom surface 2014, so that the flow passage side outer peripheral portion of the cylindrical body 2004 can be kept in the state where it is pressurized by the second pressurizing portion 2011.

However, the second pressurizing portion is not limited to the mode shown in the drawing, and it is sufficient that, by turning the operating member using the engagement portion as a fulcrum, the second pressurizing portion can pressurize the flow passage side outer peripheral portion of a portion of the cylindrical body in which the flow passage bottom member is not disposed in the interior thereof so as to completely close the flow passage of the interior of the cylindrical body. For example, the second pressurizing portion of a convex configuration, which has a height sufficient for completely closing the flow passage within the cylindrical body, may be provided on the backside of a surface of the operating member on which the first pressurizing portion is disposed. In this case, by turning the operating member in a direction opposite to the direction in which the operating member is turned upon the pressurization by using the first pressurizing portion (by turning the position of the operating member 2003 in Fig. 27 further toward the support body 2002 side), the flow passage side outer peripheral portion of a portion of the cylindrical body in which the flow passage bottom member is not disposed in the interior thereof is pressurized by the second pressurizing portion. Note that in this case, the rotating portion 2021 does not have a configuration of an oval but have a configuration of a perfect circle, so that it does not pressurize the outer peripheral portion of the cylindrical body 2004 when turned.

As in the case of the first pressurizing portion, the flow rate switcher of the present invention may have retaining means for retaining a state in which the flow passage side outer peripheral portion of the cylindrical body 2004 is pressurized by the second pressurizing portion 2011. For example, in the case where the second pressurizing portion 2011 has a structure as shown in the figure, a convex portion, which changes between a state where it protrudes from the inner side surface and a state where it is received within the inner side surface, may be formed in a site on the inner side surface of the operating member 2003 which is brought into contact with the engagement portion 2006. Then, a concave portion configured for mating engagement with this convex portion may be formed in a site on the side surface of the engagement member 2006 which is brought into contact with the operating member 2003, so that a state in which the flow passage side outer peripheral portion of the cylindrical body 2004 is pressurized by the second pressurizing portion 2011 can be retained by the mating engagement of this convex portion and the concave portion. Further, in the case where the second pressurizing portion is a pressurizing portion of a convex configuration which is provided on the backside of a surface of the operating member on which the first pressurizing portion is disposed, retaining means may be provided which has the same mechanism as that of the retaining means for retaining the first pressurizing portion in a state where it pressurizes the outer peripheral portion of the cylindrical body.

As for the materials of respective constituent members of the flow rate switcher of the present invention, the material of the cylindrical body 2004 is preferably an elastic body that deforms elastically within a certain range, such as, for example, those enumerated as examples of the material of the cylindrical body in the flow rate controllers and the flow rate regulators may be preferably used. A material preferred for the flow passage bottom member 2005 and the operating member 2003, is a material having a suitable hardness which allows those members to be molded stably. For example, those enumerated as examples of the material of the flow passage control member in the flow rate controllers and the flow rate regulators may be preferably used.

A numerical value may be printed on the operating member 2003, which indicates a fluid flow rate that is obtained when the adjustment flow passage 2032 is limited to the control groove 2008 alone upon pressurizing of the flow passage side outer peripheral portion of the cylindrical body 2002 by the first pressurizing portion 2007. In Fig. 26, such a numerical value is printed on the grip portion 2023 of the operating member 2003. Thus, the administration rate of the fluid can be learned at a glance. Note that the numerical value may be printed on the backside of the grip portion 2023.

Next, description will be made of the structure of a flow rate switcher according to the present invention incorporated into a liquid infusion set.

A soft tube having an inner peripheral length of 6.6 mm and a thickness D, shown in Fig.30, of 0.6 mm and made of polybutadiene (of JSR Co., Ltd.) was manufactured by extrusion molding as the cylindrical body 2004.

The flow passage bottom member 2005, support body 2002, and engagement portion 2006, structured as shown in Fig.27, were molded integrally from polypropylene (of Nippon Polychem Co., Ltd.).

The control groove 2008 is formed in the flow passage forming surface 2031 of the flow passage bottom member 2005. The control groove 2008 has a length of 32 mm in the axial direction of the flow passage bottom member 2005, and has such a depth and a width as to ensure a cross-sectional area of 0.05 mm². In this case, the radius R of a continuous, gentle roundness in the vicinity of the boundary 2020, in the side end portions of the flow passage bottom member 2005 as shown in Fig.30, is 0.5 mm. The radius of a concave portion formed in the center portion of the flow passage bottom member 2005, excluding the control groove 2008, is 2 mm. The flow passage bottom member 2005 has a width of 3.5 mm, an outer peripheral length of 8.4 mm, and a length of 40 mm, and the outer periphery of the section of the flow passage bottom member, 2005 excluding the control groove 2008, has a smooth surface without an edge as shown in Fig.30. As shown in Fig.26 a numerical value, indicative of a flow rate when the adjustment flow passage 2032 of the cylindrical body 2004 is limited to the control groove 2008 alone, is printed on a grip portion 2023 formed at an end portion on the downstream side of the operating member 2003.

Then, as shown in Fig.27, the end portion of the soft tube (cylindrical body 2004) is inserted, from the end portion 2030 at the downstream side of the support member 2002, which is formed integrally with the flow passage bottom member 2005 and the engagement member 2006, then passed through the support body 2002, and connected to the cylindrical body connection port 2041, provided on the downstream side of the upstream connection portion 2040 formed at the end portion on the upstream side of the support body 2002. The flow passage bottom member 2005 is inserted into an interior of the cylindrical body 2004 as shown in Fig.30. Since the peripheral length of the flow passage bottom member 2005 (8.4 mm) is longer than the peripheral length of the inner surface of the soft tube (cylindrical body 2004) (6.6 mm), a tensile strength is applied to the deformable portion 2012 of the soft tube (cylindrical body 2004) on the flow passage forming surface 2031 side, and the deformable portion 2012 is linear as shown in Fig.30. The flow passage bottom member 2005 is tapered toward the downstream to facilitate the insertion into the soft tube (cylindrical body 2004).

The operating member 2003 was manufactured using high-density polyethylene (of Nippon Polychem Co., Ltd.). As shown in Fig.27, after the soft tube (cylindrical body 2004) is inserted into the support body 2002, the substantially L-shaped engagement portion 2006 provided on the downstream side of the support body 2002 is engaged with the rotating portion 2021 formed into an oval configuration on the upstream side of the operating member 2003 to couple the support body 2002 to the operating member 2003. The second pressurizing portion 2011 is provided at the end portion on the upstream side of the rotating portion 2021. The first pressurizing portion 2007 arranged in a position between the second pressurizing portion 2011 and the grip portion 2023 on the operating member 2003 has a convex configuration and a roundness radius of 1.56 mm, and can be inserted into the concave portion of the flow passage bottom member 2005. The first pressurizing portion 2007 is set to a length of 20 mm in the axial direction.

The flow rate switcher 2001 of the present invention thus manufactured is incorporated into the line of a liquid infusion set (not shown).

Next, description will be made of how to use the flow rate switcher 2001 according to the present invention.

Fig. 27 is a sectional view showing the flushing (opening the flow passage) of the flow rate switcher 2001 of the present invention. Although not shown, the upstream side of the flow rate switcher 2001 incorporated into the liquid infusion set is connected to a liquid infusion bag and the downstream side thereof is connected to a vein needle to be inserted into a patient.

In this state, as shown in Fig.27, the operating member 2003 is first arranged substantially parallel to the cylindrical body 2004 so that the second pressurizing portion 2011 is situated on the upstream side and the grip portion 2023 is situated on the downstream side, to thereby open (flush) the flow passage in the cylindrical body 2004. As understood from Fig.27, the first pressurizing portion 2007 and the second pressurizing portion 2011 are out of operation.

Thus, when a liquid medicine is filled into the liquid infusion set and reaches the vein needle, the operating member 2003 is turned by about 90° toward an upstream side by operating the grip portion 2023, so that the operating member 2003 is situated perpendicularly to the cylindrical body 2004 and the second pressurizing portion 2011 contacts the flow passage side outer peripheral portion of the cylindrical body 2004, as shown in Fig. 28. The rotating portion 2021 situated on the upstream side of the operating member 2003 has an oval configuration, and its length in the long axial direction is the same as or slightly shorter than a length from the top surface of the substantially L-shaped engagement portion 2006 situated on the downstream side of the support body 2002, in other words, the top surface 2013 in contact with the rotating portion 2021 when the outer peripheral portion of the cylindrical body 2004 is pressurized by the second pressurizing portion 2011, to the inner surface of the support body 2002, in other words, the bottom surface 2014 to which the cylindrical portion 2004 is closely fixed. Therefore, when the operating member 2003 is turned up to the above mentioned position, the rotating portion 2021 is sandwiched between the top surface 2013 of the engagement member 2006 and the bottom surface 2014 of the support member 2002. Accordingly, the cylindrical body 2004 is pressurized by the second pressurizing portion 2011 formed at the end portion on the upstream side of the rotating portion 2021 to completely close the flow passage.

Thereafter, the vein needle is inserted into a patient. As shown in Fig.29, the operating member 2003 is further turned by about 90° toward the upstream side such that it is situated substantially parallel to the cylindrical body 2004 while the second pressurizing portion 2011 is situated on the downstream side and the grip portion 2023 is situated on the upstream side. Therefore, the flow passage side outer peripheral portion of the cylindrical body 2004 equipped with the flow passage bottom member 2005 therein is pressurized by the first pressurizing portion 2007. Accordingly, the deformable portion (deform portion) 2012 of the cylindrical body 2004 deforms and is brought into close contact with the flow passage forming surface 2031 of the flow passage bottom member 2005 with the other inner surface of the cylindrical body 2004 therebetween.

Since the section configuration, as taken perpendicularly to the axial direction of the flow passage bottom member 2005, is continuously, gently rounded to match the end portions 2020 of the flow passage bottom member 2005 as shown in Fig.30, a gap is not formed by returning force which appears at the time of pressurizing the cylindrical body 2004, and the section configurations of the close contact surfaces of the flow passage bottom member 2005 and the cylindrical body 2004 coincide excluding the control groove 2008. Therefore, the adjustment flow passage 2032 is completely closed excluding the control groove 2008.

As described above, the adjustment flow passage 2032 is limited to the control groove 2008 alone. Since the sectional area of the control groove 2008 is set to 0.05 mm² as described above, the fluid flows at a rate of 50 ml/h.

Also, in the flow rate switcher 2001, the operating member 2003 is detachable, and when a plurality of operating members which differ from one another in, the length of the first pressurizing portion 2007 in the axial direction, and the position of the first pressurizing portion are prepared, the set flow rate (velocity) of the flow rate switcher 2001 can be altered by suitably changing the operating member 2003. For instance, when the first pressurizing portion 2007 having a length of 20 mm in the axial direction of the pressurizing surface is exchanged with the first pressurizing portion 2007 having a length of 10 mm in the axial direction of the pressurizing surface, the set flow rate is changed from 50 ml/h to 100 ml/h. This is because the passage resistance generated in the control groove 2008 becomes larger as the length of the pressurizing surface of the first pressurizing portion 2007 increases.

Further, to interrupt the administration of an infusion liquid agent, the operating member 2003 is turned by 90° toward the downstream side to obtain a state shown in Fig. 28. Therefore, as mentioned above, the interior of the cylindrical body 2004 is closed by the second pressurizing portion 2011 and the bottom surface 2014 of the support body 2002.

In the case of flushing, the operating member 2003 is further turned by 90° toward a downstream side to obtain a state shown in Fig.27. As a result, the first pressurizing portion 2007 and the second pressurizing portion 2011 do not function and the flow passage is opened.

In the illustrated embodiment, when the operating member 2003 is turned toward the downstream side, the flushing is carried out and when the operating member 2003 is turned toward the upstream side, a predetermined amount of the liquid flows, but it can be composed so that, when the operating member 2003 is turned toward the upstream side, the flushing is carried out, and when the operating member 2003 is turned toward the downstream side, a predetermined amount of the liquid flows.

### INDUSTRIAL APPLICABILITY

In accordance with the present invention, the cross sectional area of the portion of the control groove covered with the soft member is varied to adjust the flow rate of a fluid to a predetermined level, so that it is possible to restrain variation with passage of time in the flow rate adjusted to a predetermined level, making it possible to perform fluid flow rate control in a stable manner. In particular, the section configurations of both side end portions of the flow passage control member are gently rounded, so that when the cylindrical body is pressurized by the pressurizing portion of the operating member, the section configurations of the close contact surface and the close contacted fixed surface of the cylindrical body and the flow passage control member coincide with each other, generating no gap other than the flow passage set by the adjustment flow passage. Thus, the flow rate set by the adjustment flow passage will not be exceeded.

Further, the inner peripheral length of the cylindrical body is set so as to be smaller than the peripheral length of the section of the flow passage control member excluding the control groove, whereby a tensile strength is applied to the deformable portion when the cylindrical body is pressurized by the pressurizing portion of the operating member, and the section configurations of the close contact surface and the close contact fixing surface of the cylindrical body and the flow passage control member further coincide with each other. Thus, no gap other than the flow passage set by the adjustment flow passage is generated, and the flow rate set by the adjustment flow passage will not be exceeded.

In the flow rate regulator, the flow rate of the fluid is adjusted to a predetermined level by changing the volume (capacity) of a site covered with a soft member forming a membrane-like portion of the cylindrical body or cylindrical member in the control groove. Accordingly, variation with passage of time in the flow rate, which has been adjusted to a predetermined level, can be restrained and the highly accurate control of the flow rate of the fluid can be carried out stably.

Particularly when the pressurizing surface of the operating member is formed into a convex configuration in accordance with the flow passage forming surface of the flow passage bottom member, the inner side at the predetermined position of the soft member can be brought into firm and close contact with the flow passage forming surface of the flow passage bottom member, thereby further improving the above mentioned effect.

Further, the limitation of part of the adjustment flow passage is canceled and the fluid can be flushed simply by pressurizing a pair of wall portions of the operating member with the finger or the like of an operator under a state where flow rate of the fluid is already adjusted, so that the operation of flushing the fluid can be carried out quickly, easily and surely.

Further, the flow rate of the fluid can be returned to the original level simply by canceling the pressurizing of the pair of wall portions of the operating member so that the pressurizing portion (pressure-closing portion) of the operating member will pressurize a predetermined position of a soft member again so that the returning operation after the flushing of the fluid can be carried out quickly, easily and surely.

Particularly when the pair of wall portions of the operating member are shaped like a convex arch projecting outward or when a side opposite to the soft member support portion of the operation support member (one of the members composing the operating member) is open, the elastic deformation function and elastic returning function of the operating member can be promoted, thereby further improving the above mentioned effect.

Further, when the soft member support portion is formed integrally with the operating member, the number of parts of the flow rate regulator can be reduced, thereby making it possible to simplify the structure of the flow rate regulator and cut the cost of the flow rate regulator.

According to the flow rate switcher of the present invention, without the burdening work of adjusting the flow rate, a fluid can be administered at a stable rate which does not vary with passage of time by simple operation and the interruption of the administration of the fluid and the flush operation of the fluid can be carried out.

## Claims

1. A flow rate switcher comprising:
a support body (2002) having an engagement portion (2006);
an operating member (2003) which is engaged such that it can be turned using the engagement portion (2006) as a fulcrum, wherein the operating member has a first pressurizing portion (2007) for pressurizing the flow passage side outer peripheral portion of a cylindrical body (2004); and
said cylindrical body (2004) situated between the support body (2002) and the operating member (2003), whose outer periphery is partially supported by the support body (2002), and which has an inlet and an outlet for a fluid, **characterized by**
a flow passage bottom member (2005) which is formed of a hard material and is in close contact with and in part closely fixed to the support body side of an interior of the cylindrical body (2004) forming a flow passage for the fluid, wherein:
the operating member has a second pressurizing portion (2011) for pressurizing the flow passage side outer peripheral portion of the cylindrical body (2004);
the flow passage bottom member (2.005) has a control groove (2008) extending in an axial direction of the cylindrical body (2004); and
in the vicinity of a boundary between the cylindrical body (2004) and the flow passage bottom member (2005), a section configuration of both side end portions of the flow passage bottom member (2005) exhibits a continuous, gentle roundness, and that of a center thereof portion (2031) exhibits a concave configuration.

2. The flow rate switcher according to claim 1, wherein: the first pressurizing portion (2007) is arranged in a position where, at the time of pressurizing, it pressurizes the flow passage side outer peripheral portion of the cylindrical member in cooperation with the flow passage bottom member (2005); and the second pressurizing portion (2011) is arranged in a position where, at the time of pressurizing, it pressurizes the flow passage side outer peripheral portion of the cylindrical member in cooperation with the support member.

3. The flow rate switcher according to claim 1 or 2, wherein: the first pressurizing portion (2007) has a convex section configuration of a size which allows insertion thereof into the concave configuration; and a radius R of the continuous, gentle roundness of the section configuration of the both side end portions of the flow passage control member and a thickness D of the cylindrical body (2004) are in the relationship: R ≥ D/10.

4. The flow rate switcher according to any one of claims 1 to 3, wherein a circumferential length of an interior of the cylindrical body (2004) is set to be smaller than a peripheral length of the section of the flow passage bottom member (2005).

5. The flow rate switcher according to any one of claims 1 to 4, wherein: the cylindrical body (2004) has a deform portion in which a pressurized site deforms at the time of pressurization; the deformed deform portion and the flow passage bottom member (2005) are in close contact with each other excluding the control groove; the deform portion is constantly under a tensile strength in the inner side of the section taken perpendicularly to the axial direction of the cylindrical body (2004); in a non-pressurized state, the section configuration of the deform portion as taken perpendicularly to the axial direction of the cylindrical body (2004) is linear; and at least a surface where the interior of the cylindrical body (2004) and the flow passage bottom member (2005) comes into contact with each other in the vicinity of a flow passage formed by the deform portion and the flow passage bottom member (2005) is a close contact portion that is not adhesion fixed.

6. The flow rate switcher according to any one of claims 1 to 5, in which a numerical value indicative of a flow rate is described on the operating member (2003).

7. The flow rate switcher according to any one of claims 1 to 6, wherein the cylindrical body (2004) is either one of a liquid, transfusion tube, a blood transfusion tube, and a nutritional transfusion tube.

## Patentansprüche

1. Strömungsratenumschalteinrichtung, mit:
einem Stützkörper (2002) mit einem Eingriffsabschnitt (2006);
einem Betätigungsbauteil (2003), das derart in Eingriff ist, dass es unter Verwendung des Eingriffsabschnitts (2006) als ein Drehpunkt gedreht werden kann, wobei das Betätigungsbauteil einen ersten Druckbeaufschlagungsabschnitt (2007) zum Beaufschlagen des strömungsdurchgangsseitigen Außenumfangsabschnitts eines zylindrischen Körpers (2004) mit Druck aufweist, und
dem zylindrischen Körper (2004), der sich zwischen dem Stützkörper (2002) und dem Betätigungsbauteil (2003) befindet, dessen Außenumfang durch den Stützkörper (2002) teilweise gestützt ist, und der einen Einlass und einen Auslass für ein Fluid hat,
**gekennzeichnet, durch**
ein Strömungsdurchgangsbodenbauteil (2005), das aus einem harten Material ausgebildet ist und in engem Kontakt ist mit und zum Teil fest mit der Stützkörperseite eines Inneren des zylindrischen Körpers (2004) befestigt ist, der einen Strömungsdurchgang für das Fluid bildet, wobei:
das Betätigungsbauteil einen zweiten Druckbeaufschlagungsabschnitt (2011) zum Beaufschlagen des strömungsdurchgangsseitigen Außenumfangsabschnitts des zylindrischen Körpers (2004) mit Druck hat;
das Strömungsdurchgangsbodenbauteil (2005) eine Steuerungsnut (2008) hat, die sich in einer axialen Richtung des zylindrischen Körpers (2004) erstreckt; und
eine Querschnittskonfiguration von beiden Seitenendabschnitten des Strömungsdurchgangsbodenbauteils (2005) in der Nähe einer Grenze zwischen dem zylindrischen Körper (2004) und dem Strömungsdurchgangsbodenbauteil (2005) eine stetige, leichte Rundung aufweist, und ein Mittelabschnitt (2031) von diesem eine konkave Konfiguration aufweist.

2. Strömungsratenumschalteinrichtung nach Anspruch 1, wobei:
der erste Druckbeaufschlagungsabschnitt (2007) an einer Position angeordnet ist, an der er zu dem Zeitpunkt eines Beaufschlagens mit Druck den strömungsdurchgangsseitigen Außenumfangsabschnitt des zylindrischen Bauteils zusammen mit dem Strömungsdurchgangsbodenbauteil (2005) mit Druck beaufschlagt; und
der zweite Druckbeaufschlagungsabschnitt (2011) an einer Position angeordnet ist, an der er zu dem Zeitpunkt eines Beaufschlagens mit Druck den strömungsdurchgangsseitigen Außenumfangsabschnitt des zylindrischen Bauteils zusammen mit dem Stützbauteil mit Druck beaufschlagt.

3. Strömungsratenumschalteinrichtung nach Anspruch 1 oder 2, wobei:
der erste Druckbeaufschlagungsabschnitt (2007) eine konvexe Querschnittskonfiguration einer Größe hat, die ein Einsetzen von diesem in die konkave Konfiguration ermöglicht; und ein Radius R der stetigen, leichten Rundung der Querschnittskonfiguration der beiden Endabschnitte des Strömungsdurchgangssteuerbauteils und eine Dicke D des zylindrischen Körpers (2004) in der Beziehung: R ≥ D/10 stehen.

4. Strömungsratenumschalteinrichtung nach einem der Ansprüche 1 bis 3, wobei eine Umfangslänge eines Inneren des zylindrischen Körpers (2004) eingestellt ist, um kleiner als eine Umfangslänge des Querschnitts des Strömungsdurchgangsbodenbauteils (2005) zu sein.

5. Strömungsratenumschalteinrichtung nach einem der Ansprüche 1 bis 4, wobei: der zylindrische Körper (2004) einen Deformationsabschnitt hat, in dem sich eine mit Druck beaufschlagte Stelle zu dem Zeitpunkt einer Druckbeaufschlagung deformiert; der deformierte Deformationsabschnitt und das Strömungsdurchgangsbodenbauteil (2005) mit Ausnahme der Steuerungsnut in engem Kontakt miteinander sind; der Deformationsabschnitt konstant unter einer Zugspannung an der Innenseite des Querschnitts ist, der senkrecht zu der axialen Richtung des zylindrischen Körpers (2004) genommen ist; wobei die Querschnittskonfiguration des Deformationsabschnitts in einem nicht druckbeaufschlagten Zustand, senkrecht zu der axialen Richtung des zylindrischen Körpers (2004) genommen, linear ist; und wenigstens eine Fläche, an der das Innere des zylindrischen Körpers (2004) und das Strömungsdurchgangsbodenbauteil (2005) in der Nähe eines Strömungsdurchgangs, der durch den Deformationsabschnitt und das Strömungsdurchgangsbodenbauteil (2005) ausgebildet ist, in Kontakt miteinander kommen, ein Abschnitt eines engen Kontakts ist, der nicht durch Adhäsion befestigt ist.

6. Strömungsratenumschalteinrichtung nach einem der Ansprüche 1 bis 5, in der ein numerischer Wert, der eine Strömungsrate anzeigt, auf dem Betätigungsbauteil (2003) dargestellt ist.

7. Strömungsratenumschalteinrichtung nach einem der Ansprüche 1 bis 6, wobei der zylindrische Körper (2004) eines von einem Flüssigkeitstransfusionsschlauch, einem Bluttransfusionsschlauch und einem Ernährungstransfusionsschlauch ist.

## Revendications

1. Régulateur de débit comprenant :
un corps de support (2002) comportant une partie d'accrochage (2006) ;
un élément d'actionnement (2003) qui est engagé de telle manière qu'il peut être tourné en utilisant la partie d'accrochage (2006) comme point d'appui, l'élément d'actionnement comportant une première partie de mise en pression (2007) pour mettre en pression la partie périphérique extérieure côté passage d'écoulement d'un corps cylindrique (2004) ; et
ledit corps cylindrique (2004) étant situé entre le corps de support (2002) et l'élément d'actionnement (2003), dont la périphérie extérieure est partiellement supportée par le corps de support (2002), et qui comporte une entrée et une sortie pour un fluide,
**caractérisé par** un élément inférieur de passage d'écoulement (2005) qui est fait d'un matériau dur et qui est en contact étroit avec et partiellement fixé étroitement au côté corps de support d'une partie intérieure du corps cylindrique (2004) formant un passage d'écoulement pour le fluide, dans lequel :
l'élément d'actionnement comporte une deuxième partie de mise en pression (2011) pour mettre en pression la partie périphérique extérieure côté passage d'écoulement du corps cylindrique (2004) ;
l'élément inférieur de passage d'écoulement (2005) comporte une rainure de contrôle (2008) s'étendant dans une direction axiale du corps cylindrique (2004) ; et
au voisinage d'une limite entre le corps cylindrique (2004) et l'élément inférieur de passage d'écoulement (2005), une configuration de section des deux parties d'extrémités latérales de l'élément inférieur de passage d'écoulement (2005) présente une rondeur douce et continue, et celle d'une partie centrale (2031) présente une configuration concave.

2. Régulateur de débit selon la revendication 1, dans lequel la première partie de mise en pression (2007) est placée dans une position où, au moment de la mise en pression, elle met en pression la partie périphérique extérieure côté passage d'écoulement de l'élément cylindrique en coopération avec l'élément inférieur de passage d'écoulement (2005), et la deuxième partie de mise en pression (2011) est placée dans une position où, au moment de la mise en pression, elle met en pression la partie périphérique extérieure côté passage d'écoulement de l'élément cylindrique en coopération avec l'élément de support.

3. Régulateur de débit selon la revendication 1 ou 2, dans lequel la première partie de mise en pression (2007) a une configuration de section convexe d'une taille qui permet son insertion dans la configuration concave, et un rayon R de la rondeur douce et continue de la configuration de section des deux parties d'extrémités latérales de l'élément de commande de passage d'écoulement et une épaisseur D du corps cylindrique (2004) qui vérifient la relation : R ≥ D/10.

4. Régulateur de débit selon l'une quelconque des revendications 1 à 3, dans lequel une longueur circonférentielle d'une partie intérieure du corps cylindrique (2004) est plus petite qu'une longueur périphérique de la section de l'élément inférieur de passage d'écoulement (2005).

5. Régulateur de débit selon l'une quelconque des revendications 1 à 4, dans lequel le corps cylindrique (2004) comporte une partie déformable dans laquelle un site sous pression se déforme au moment de la mise en pression, la partie déformable déformée et l'élément inférieur de passage d'écoulement (2005) sont en contact étroit l'un avec l'autre, à l'exclusion de la rainure de contrôle, la partie déformable est constamment sous une force de traction dans le côté intérieur de la section prise perpendiculairement à la direction axiale du corps cylindrique (2004), dans un état non pressurisé, la configuration de section de la partie déformable prise perpendiculairement à la direction axiale du corps cylindrique (2004) est linéaire, et au moins une surface où l'intérieur du corps cylindrique (2004) et l'élément inférieur de passage d'écoulement (2005) viennent au contact l'un de l'autre au voisinage d'un passage d'écoulement formé par la partie déformable et l'élément inférieur de passage d'écoulement (2005) est une partie de contact étroit qui n'est pas fixée par adhésion.

6. Régulateur de débit selon l'une quelconque des revendications 1 à 5, dans lequel une valeur numérique représentant un débit est affichée sur l'élément d'actionnement (2003).

7. Régulateur de débit selon l'une quelconque des revendications 1 à 6, dans lequel le corps cylindrique (2004) est soit un tube de transfusion de liquide, soit un tube de transfusion sanguine, soit un tube de transfusion nutritionnelle.
